# FASCICULE DE BREVET EUROPEEN

(11) **EP 3 129 784 B1**
(45) Date de publication et mention de la délivrance du brevet: **18.12.2019**
(21) Numéro de dépôt: 15714824.8
(22) Date de dépôt: 08.04.2015
(51) Int. Cl.: G01N 33/543

(54) **UTILISATION D'UN COLORANT POUR AMÉLIORER LA DÉTECTION DU SIGNAL DANS UN PROCÉDÉ D'ANALYSE**
VERWENDUNG EINES FARBSTOFFS ZUR VERBESSERUNG DER SIGNAL-DETEKTION IN EINEM ANALYSEVERFAHREN
USE OF A DYE FOR IMPROVING SIGNAL DETECTION IN A METHOD OF ANALYSIS

(30) Priorité: 09.04.2014 FR 1453168
(43) Date de publication de la demande: 15.02.2017
(73) Titulaire: Bio-Rad Europe GmbH, 4051 Basel (CH)
(72) Inventeur: MERANDON, Bertrand, F-92320 Chatillon (FR); VEDRINE, Christophe, F-92400 Courbevoie (FR)
(74) Mandataire: Lavoix
(86) Numéro de dépôt international: PCT/EP2015/057633
(87) Numéro de publication internationale: WO 2015/155248

(56) Documents cités:
- US-A- 5 082 768
- US-A1- 2001 006 820
- US-A1- 2010 267 071
- PETERSEN J. ET AL.: "Comparison of absorbance and fluorescence methods for determining liquid dispensing precision", J. ASS. LAB. AUTOM., vol. 10, no. 2, avril 2005 (2005-04), pages 82-87, XP027677891,
- AULD D.S. ET AL.: "Characterization of chemical libraries for luciferase inhibitory activity", J. MED. CHEM., vol. 51, no. 8, avril 2008 (2008-04), pages 2372-2386, XP055155041,

## Description

### Domaine technique

La présente invention concerne l'amélioration de la détection d'un signal correspondant à la présence d'un analyte lors d'un procédé d'analyse, en particulier lorsque le procédé d'analyse nécessite une acquisition du signal en présence d'une phase liquide.

### Etat de la technique

Un procédé d'analyse permet de détecter la présence éventuelle d'un ou plusieurs analytes dans un échantillon. Un procédé d'analyse est généralement réalisé sur un support solide. Parmi les procédés d'analyse, le procédé d'analyse multiplexe permet de détecter, de manière simultanée, la présence éventuelle de plusieurs analytes au sein d'un même échantillon. Un procédé d'analyse multiplexe peut être réalisé sur un support solide comprenant des spots ou encore un ensemble de billes.

Classiquement, un procédé d'analyse comprend une étape de mise en présence d'un échantillon à analyser avec au moins un spot d'un support solide ou des billes comprenant un ligand de capture spécifique d'un analyte à détecter, une étape d'ajout d'un ligand de détection spécifique d'un analyte à détecter et couplé à un marqueur direct ou indirect, une éventuelle étape de révélation par ajout d'un rapporteur lui-même couplé à un marqueur direct ou indirect, et une étape de détection du signal (appelée également étape d'acquisition du signal). En cas de marqueur indirect de type enzyme peroxydase, l'ajout d'un substrat de l'enzyme permet une réaction enzymatique qui conduit à la production d'un composé chimiluminescent. Le signal est alors détecté en chimiluminescence.

La détection d'un signal en chimiluminescence nécessite, en principe, de réaliser l'acquisition du signal en présence du substrat de l'enzyme, c'est-à-dire en présence d'une phase liquide, afin de permettre la production du composé chimiluminescent en continu. En effet, si une étape de lavage est effectuée avant l'acquisition du signal, le substrat résiduel est éliminé et la réaction enzymatique s'arrête. Or, le signal émis par le composé chimiluminescent s'éteint progressivement. En conséquence, le substrat de l'enzyme doit être présent dans une phase liquide au contact de la phase solide pour permettre une émission de signal suffisamment stable et reproductible.

Toutefois, l'acquisition d'un signal au niveau des spots d'un puits d'une microplaque en présence d'une phase liquide conduit à des interférences lumineuses. Ces interférences lumineuses ont plusieurs origines : d'une part, des photons émis à partir des spots vers le haut du puits peuvent interagir avec les composés de la solution comprenant le composé chimiluminescent et être diffusés dans toutes les directions ; d'autre part, les photons peuvent également être réfléchis par les parois du puits et par le changement de milieu au niveau de l'interface liquide / air, plus précisément au niveau du ménisque formé par l'interaction de la paroi du puits et de la solution comprenant le composé chimiluminescent.

Ces interférences lumineuses peuvent produire des spots dits « spots jumeaux », légèrement décalés par rapport aux spots réels, un anneau lumineux visible en périphérie du puits, voire un arc lumineux lorsque le signal émis au niveau d'un spot est fort. Ces interférences lumineuses induisent donc un bruit de fond problématique, qui peut être à l'origine de résultats faussement négatifs ou de résultats faussement positifs. Par exemple, l'anneau lumineux autour du puits peut biaiser le seuil du bruit de fond, un faible signal se trouvant alors noyé dans le bruit de fond. Les interférences lumineuses peuvent également gêner la vérification de l'absence de défaut d'un spot effectuée par une mesure annulaire autour d'un spot.

Le document EP 0 165 072 décrit l'utilisation d'un atténuateur, de préférence un mélange de colorants rouge et jaune, pour supprimer la lumière indésirable et les résultats faussement positifs dans un procédé impliquant une émission de lumière luminescente. Dans ce document, la lumière indésirable est adjacente aux surfaces mesurées. Le test est réalisé dans une pipette allongée creuse comprenant une pluralité de fils de coton, chaque fil de coton étant lié à un seul type d'antigène. La pipette est fermée avec une fenêtre transparente. Le signal est révélé dans l'obscurité en pressant un film à la surface de la pipette. L'atténuateur permet de supprimer les résultats faussement positifs liés à la liaison non spécifique de l'enzyme peroxydase HRP sur les fils de coton.

Le document US 8 163 562 décrit un test permettant de réduire la lumière indésirable résultant de la fluorescence d'une solution dans laquelle baigne un compartiment cellulaire qui est, de préférence, une cellule. Cette fluorescence indésirable provient notamment des sondes ou composés chimiques utilisés pendant le test. Le signal à détecter provient quant à lui d'un agent producteur de photons situé dans le compartiment membranaire. A cet effet, un agent réducteur de photons imperméable à la membrane et ne se liant pas spécifiquement à la membrane est utilisé dans la solution aqueuse en contact avec la surface externe du compartiment membranaire. L'agent réducteur de photons peut être un colorant ou un mélange de colorants.

Il existe donc un besoin de solutions permettant d'améliorer la détection d'un signal correspondant à la présence d'un analyte dans le cadre d'un procédé d'analyse sur spot(s) au cours duquel l'acquisition du signal au niveau du ou des spots a lieu en présence d'une phase liquide, afin de sécuriser les résultats obtenus en évitant des résultats faussement positifs ou faussement négatifs.

### Description détaillée

De manière surprenante, dans un procédé d'analyse sur spot(s), les inventeurs ont montré que l'utilisation d'un colorant, par exemple la tartrazine, permet de supprimer, partiellement ou en totalité, les interférences lumineuses indésirables qui se produisent ordinairement lors de l'acquisition d'un signal en présence d'une phase liquide, sans toutefois interférer ou en interférant peu avec l'intensité du signal détecté correspondant à la présence d'un analyte au niveau d'un spot (par exemple en luminescence et/ou en fluorescence), ni avec l'éventuelle détection de la fluorescence émise par un fluorophore présent en tant que contrôle au niveau du ou des spots. L'utilisation d'un colorant selon l'invention permet ainsi de sécuriser les résultats obtenus à l'issue d'un procédé d'analyse, c'est-à-dire de garantir la fiabilité desdits résultats obtenus à l'issue dudit procédé, en particulier en évitant de rendre des résultats faussement positifs (appelés également « faux positifs ») et/ou faussement négatifs (appelé également « faux négatifs »).

Un « faux positif » est un résultat positif traduisant la présence d'un ou de plusieurs analytes à détecter dans un échantillon, alors que ledit ou lesdits analytes n'étaient présents dans l'échantillon et n'auraient donc pas dû être détectés.

Un « faux négatif » est un résultat négatif traduisant l'absence d'un ou de plusieurs analytes à détecter dans un échantillon, alors que ledit ou lesdits analytes étaient présents dans l'échantillon et auraient dû être détectés.

Le signal détecté correspondant à la présence d'un analyte au niveau d'un spot permet de détecter la présence d'un analyte dans un échantillon et/ou de quantifier ledit analyte dans ledit échantillon.

Le signal détecté correspondant à la présence d'un analyte au niveau d'un spot est un rayonnement électromagnétique, en particulier une émission lumineuse.

Le signal détecté correspondant à la présence d'un analyte au niveau d'un spot est, de préférence, un signal détecté en luminescence, par exemple en chimiluminescence, et/ou un signal détecté en fluorescence.

Le fluorophore présent dans le ou les spots d'un support solide peut servir, entre autres, à contrôler la qualité du ou des spots (en particulier leur présence, localisation et/ou intégrité) à l'issue d'un procédé d'analyse et/ou à améliorer la sensibilité de la détection du ou des analytes en définissant une grille de lecture du signal correspondant au(x) analyte(s) à partir de la localisation réelle du ou des spots à la fin du procédé d'analyse. L'utilisation d'un fluorophore dans le ou les spots d'un support solide permet donc également de sécuriser les résultats d'un procédé d'analyse sur spot(s).

L'utilisation d'un colorant selon l'invention permet ainsi d'améliorer (et donc de sécuriser) la détection d'un signal correspondant à la présence d'un analyte lors d'un procédé d'analyse, en masquant et/ou absorbant, partiellement ou en totalité, les interférences lumineuses indésirables lors de l'acquisition d'un signal à détecter en présence d'une phase liquide. L'amélioration de la détection du signal peut être évaluée par une mesure du rapport « signal détecté sur bruit de fond ».

Par ailleurs, l'utilisation d'un colorant dans un procédé d'analyse selon l'invention présente l'avantage de pouvoir utiliser un support solide dont la ou les parois du ou des compartiments comprennent ou sont constituées d'un matériau transparent, ce type de support étant moins coûteux que ceux comprenant ou étant constitués d'un matériau opaque.

Un premier objet de l'invention est ainsi de fournir un procédé d'analyse multiplexe, permettant d'améliorer la détection d'un signal correspondant à la présence d'un analyte comprenant les étapes suivantes :
a) fournir un support solide comprenant au moins deux compartiments, lesdits compartiments comprenant au moins deux spots destinés à la détection d'un analyte,
b) mettre un échantillon à analyser en présence des spots d'un compartiment du support solide,
c) mettre en présence des spots dudit compartiment au moins un ligand de détection d'un analyte, ledit ligand de détection d'un analyte étant couplé à un marqueur de détection direct ou indirect,
d) lorsque ledit marqueur de détection est un marqueur indirect, mettre en présence des spots dudit compartiment un rapporteur du marqueur de détection indirect couplé audit ligand de détection,
e) lorsque le rapporteur utilisé à l'étape d) est couplé à un marqueur indirect, mettre en présence des spots dudit compartiment un rapporteur du marqueur de détection indirect couplé audit rapporteur,
f) mettre au moins un colorant en présence des spots dudit compartiment, ledit ou lesdits colorants étant compris dans une phase liquide en contact des spots dudit compartiment, et ledit au moins un colorant étant la tartrazine, et
g) détecter un signal correspondant à la présence d'un analyte au niveau du ou des spots dudit compartiment, en présence de la phase liquide comprenant ledit ou lesdits colorants,
ledit procédé comprenant en outre une ou plusieurs étapes de lavage permettant d'éliminer les composés non liés aux spots ou aux différents composés liés directement ou indirectement aux spots,
aucune étape de lavage n'étant effectuée entre l'étape f) et l'étape g).

Un deuxième objet de l'invention concerne l'utilisation d'au moins un colorant dont au moins la tartrazine pour améliorer la détection d'un signal correspondant à la présence d'un analyte dans un procédé d'analyse sur spots, l'amélioration de la détection du signal étant par exemple caractérisée par une diminution de l'intensité du bruit de fond.

### Echantillon

L'échantillon à analyser est de préférence un échantillon biologique.

L'échantillon biologique peut être un fluide biologique, tel qu'un échantillon de sang, dérivé de sang (tel que plasma ou sérum), urine, fluide céphalorachidien, salive, ou un échantillon de tissu, tel qu'un tissu obtenu par biopsie, une cellule un ensemble de cellules, un extrait végétal, ou leurs combinaisons.

Un dérivé de sang désigne tout produit, en particulier fluide, obtenu à partir d'un échantillon de sang.

L'échantillon à analyser peut également être un milieu de culture et/ou un surnageant de culture.

Avant d'être analysé, l'échantillon peut subir une ou plusieurs étapes préalables de traitement, tels que dilution, centrifugation, traitement thermique, lyse cellulaire (par exemple par un ou plusieurs agents chaotropiques, un ou plusieurs agents réducteurs et/ou par chauffage), extraction, réaction de PCR (*Polymerase Chain Reaction*), ajout d'un ligand de détection non marqué ou leurs combinaisons. L'ajout d'un ligand de détection non marqué est en particulier utile pour la mise en œuvre d'un test de neutralisation, qui est en soi un test connu de l'homme du métier.

L'échantillon peut également être un mélange d'au moins deux échantillons qui peuvent être de même nature ou de nature différente.

A titre d'exemple de mélange d'échantillons de nature différente, on peut citer un mélange de sang et de sérum, un mélange de sang et de plasma, un mélange de sérum et de plasma, ou encore un mélange de sang, sérum et plasma.

Un échantillon préféré selon l'invention est un échantillon ou un mélange d'échantillons de sang et/ou dérivé du sang.

### Analvte

Un analyte à détecter dans l'échantillon peut être tout type de composé, naturel ou synthétique, que l'on souhaite détecter et / ou quantifier dans un échantillon.

Un analyte peut par exemple être une protéine, un peptide, une glycoprotéine, un glucide, un lipide, une cellule, une organelle, un virus ou un acide nucléique.

La cellule peut être une cellule animale, une cellule végétale, une cellule de bactérie, une cellule de métazoaire, une cellule de levure, une cellule de champignon ou un protozoaire.

Un acide nucléique désigne un polymère de nucléotides reliés par des liaisons phosphodiester, tel qu'un acide désoxyribonucléique (ADN), un acide ribonucléique (ARN) ou un analogue de ceux-ci, tels que des phosphorothioates ou des thioesters, sous forme simple brin ou double brin.

Un analyte ou au moins un des analytes est par exemple choisi dans le groupe consistant en un antigène, un anticorps, un fragment d'anticorps, un haptène, une hormone, un récepteur d'hormone, une enzyme ou un acide nucléique.

Par « antigène », on désigne ici une molécule naturelle ou synthétique reconnue par des anticorps ou des cellules du système immunitaire et capable d'induire une réponse immunitaire. Un antigène est par exemple une protéine, un peptide, une glycoprotéine, un glucide ou un lipide.

Par « haptène », on désigne ici une molécule de faible poids moléculaire capable d'être reconnue par le système immunitaire, mais qui est immunogène uniquement lorsqu'elle est couplée à une molécule porteuse.

Par « molécule porteuse» (ou « molécule support »), on entend notamment dans la présente demande une molécule porteuse protéique ou glucidique. Une molécule porteuse peut être un polypeptide (en particulier une protéine ou un peptide) naturel ou non naturel (par exemple une protéine recombinante ou un peptide synthétique), un polymère fonctionnalisé (de type dextran, polysaccharide ou poly-lysine), un co-polymère mixte (en particulier un co-polymère d'acides aminés différents, par exemple un co-polymère lysine-tyrosine) ou un anticorps (en particulier un anticorps monoclonal ou un anticorps polyclonal), par exemple une immunoglobuline (appelée également Ig). Un exemple de molécule porteuse est la BSA (albumine sérique bovine).

Un analyte ou au moins un des analytes est, de préférence, un composé permettant de diagnostiquer une condition d'un sujet, pathologique ou non, ou de diagnostiquer les risques de développer une condition, pathologique ou non. Un exemple de condition non pathologique est une grossesse.

Le sujet peut être un homme, un animal non humain ou un végétal. L'animal non humain est de préférence un mammifère, tel qu'un chat, chien, singe, lapin, souris, rat.

Le terme « homme » est utilisé de manière large et désigne notamment un homme ou une femme de tout âge, tel qu'un nourrisson, un enfant, un adolescent, un adulte ou une personne âgée.

Lorsque l'analyte ou au moins un des analytes est un antigène, il s'agit de préférence d'un antigène permettant de diagnostiquer une infection, par exemple une infection causée par un virus, une bactérie, un champignon ou un parasite.

Lorsque l'analyte ou au moins un des analytes est un anticorps, il s'agit de préférence d'un anticorps permettant de diagnostiquer une infection, par exemple une infection causée par un virus, une bactérie, un champignon ou un parasite.

Typiquement, il peut s'agir d'un ou plusieurs antigène(s) et/ou d'un ou plusieurs anticorps spécifique(s) de :
- un virus, tel que VIH (*Virus de l'Immunodéficience Humaine),* en particulier VIH-1 ou VIH-2, VHB (*Virus de l'Hépatite B*), VHC (*Virus de l'Hépatite C*), VPH (*papillomavirus humain*), HTLV (*Virus T-lymphotropique humain*), en particulier HTLV-I ou HTLV-II,
- un parasite, tel qu'un parasite susceptible de provoquer la Toxoplasmose (en particulier *Toxoplasma gondii*), la Malaria (en particulier un parasite du genre *Plasmodium,* par exemple *Plasmodium falciparum, Plasmodium vivax, Plasmodium ovale, Plasmodium malariae* ou *Plasmodium knowlesi*) ou la maladie de Chagas (en particulier *Trypanosoma cruzi*), chez un homme ou un animal non humain, ou
- une bactérie, telle qu'une bactérie susceptible de provoquer la Syphilis (en particulier *Treponema pallidum*) ou la maladie de Lyme (en particulier une bactérie du genre *Borrelia*), chez un homme ou un animal non humain.

Par « parasite », on désigne ici un métazoaire ou un protozoaire parasitant un organisme et entraînant une parasitose. Un parasite au sens de l'invention n'est donc ni un virus, ni une bactérie, ni un champignon.

L'analyte ou au moins un des analytes peut également être un marqueur d'une maladie, tel qu'un marqueur d'une maladie cardiovasculaire ou un marqueur du diabète, un marqueur de l'évolution d'une maladie, telle qu'une hépatite, un marqueur de l'évolution d'une infection causée par un virus, une bactérie, un champignon ou un parasite, un marqueur de résistance à un traitement, par exemple à un traitement antiviral, un traitement antibiotique ou un traitement contre un cancer.

Plusieurs (par exemple deux, trois, quatre, cinq, six, sept, huit, neuf, dix, onze, douze, treize, quatorze, quinze, seize ou plus de seize) analytes tels que décrits dans la présente demande peuvent être détectés simultanément dans un échantillon au cours d'un procédé d'analyse multiplexe. Cela peut permettre de diagnostiquer, dans un même échantillon, une ou plusieurs infection(s) ou maladie(s), l'évolution d'une infection ou maladie, une condition (pathologique ou non), un risque de développer une condition (pathologique ou non) ou encore un marqueur de résistance à un traitement, chez un sujet.

Les analytes détectés au cours d'un procédé d'analyse multiplexe peuvent être de même nature (par exemple uniquement des anticorps ou uniquement des antigènes) ou de nature différente (par exemple, au moins un antigène et au moins un anticorps).

### Ligand de capture

Un ligand de capture est un composé fixé sur un support solide au niveau d'un spot.

Au moins un ligand de capture est spécifique d'un analyte à détecter dans l'échantillon.

Un ligand de capture peut être un anticorps, un antigène, un peptide, un glucide, un lipide ou un acide nucléique.

Un ligand de capture est, de préférence, un anticorps ou un antigène.

Lorsqu'un ligand de capture est un anticorps, il s'agit par exemple d'un anticorps monoclonal ou d'un anticorps polyclonal.

### Ligand de détection

Un ligand de détection est destiné à révéler la présence d'un composé dont il est spécifique.

Un ligand de détection peut être un anticorps, un antigène, un peptide, un glucide, un lipide ou un acide nucléique.

Un ligand de détection est, de préférence, un anticorps ou un antigène.

Lorsqu'un ligand de détection est un anticorps, il s'agit par exemple d'un anticorps monoclonal ou d'un anticorps polyclonal.

Un ligand de détection est, de préférence, un ligand de détection marqué, c'est-à-dire un ligand auquel est attaché un marqueur de détection, de manière covalente ou non.

Lorsqu'un ligand de détection n'est pas marqué, sa détection peut être obtenue en utilisant un anticorps marqué spécifique dudit ligand de détection.

Au moins un ligand de détection est spécifique d'un analyte à détecter dans l'échantillon.

Un ligand de détection peut être identique au ligand de capture ou à un des ligands de capture utilisés, à l'exception de la présence éventuelle d'un marqueur de détection, et/ou se lier au composé dont il est spécifique au niveau de la même zone que celle liée par le ligand de capture ou un des ligands de capture. Dans ce cas, si ledit ligand de capture et ledit ligand de détection sont des anticorps, il s'agit alors d'un « sandwich homologue ».

Un ligand de capture et le ligand de détection ou un des ligands de détection peuvent être spécifiques de zones distinctes au niveau du composé dont ils sont spécifiques, de manière à éviter une compétition du ligand de capture et du ligand de détection vis-à-vis du composé dont ils sont spécifiques, en raison d'un encombrement stérique. Dans ce cas, si ledit ligand de détection et ledit ligand de capture sont des anticorps, il s'agit alors d'un « sandwich hétérologue ».

Dans un mode de réalisation préféré, un ligand de détection et un ligand de capture spécifiques du même composé ne se lient pas au même endroit sur ledit composé. Plus préférentiellement, ledit ligand de détection se lie à une zone dudit composé qui est éloignée de la zone de liaison avec ledit ligand de capture.

Dans un autre mode de réalisation préféré, un ligand de détection est identique à un ligand de capture, à l'exception de la présence éventuelle d'un marqueur de détection, et/ou se lie au composé dont il est spécifique au niveau de la même zone que celle liée par ledit ligand de capture, lorsque le composé dont il est spécifique est sous forme d'un complexe présentant au moins deux zones de liaison identiques.

### Marqueur de détection

Un marqueur de détection peut être un marqueur direct ou un marqueur indirect.

Un marqueur direct est un marqueur dont le signal peut être détecté directement, c'est-à-dire sans nécessiter l'ajout préalable d'un rapporteur.

Un marqueur direct est, par exemple, sélectionné dans le groupe consistant en un fluorophore, un composé luminescent, des nanoparticules fluorescentes ou luminescentes.

Un composé « luminescent» peut être un composé électroluminescent, un composé thermoluminescent ou un composé chimiluminescent. Dans un mode de réalisation préféré, le composé luminescent est un composé chimiluminescent.

Un exemple de composé luminescent (plus précisément de composé thermoluminescent) que l'on peut utiliser comme marqueur direct consiste en des nanoparticules de silice comprenant (par exemple additionnée de ou dopée par (de l'anglais « doped »)) des molécules d'un composé dioxétane, en particulier le composé 1,2-dioxétane, ou d'un dérivé d'un composé dioxétane, par exemple un dérivé du 1,2-dioxétane.

Un marqueur indirect est un marqueur dont la détection du signal nécessite préalablement l'ajout d'un rapporteur (appelé également premier rapporteur) et, si ledit rapporteur est lui-même couplé à un marqueur de détection indirect, l'ajout d'un deuxième rapporteur du marqueur de détection indirect couplé audit premier rapporteur.

Un marqueur indirect est, par exemple, sélectionné dans le groupe consistant en une enzyme, un ligand d'un couple ligand-récepteur, un récepteur d'un couple ligand-récepteur, un haptène, un antigène et un anticorps.

Un ligand ou un récepteur d'un couple ligand-récepteur est, par exemple, la biotine, un analogue de la biotine, l'avidine, la streptavidine, la neutravidine ou la digoxigénine.

Un rapporteur est un substrat d'un marqueur indirect ou une molécule se liant spécifiquement à un marqueur indirect, ladite molécule étant elle-même un marqueur direct ou indirect ou étant elle-même couplée à un marqueur direct ou indirect.

Un substrat est, par exemple, le substrat d'une enzyme.

Une molécule se liant spécifiquement à un marqueur indirect est, par exemple, un ligand ou un récepteur d'un couple ligand-récepteur, tel que la biotine, un analogue de la biotine, l'avidine, la streptavidine, la neutravidine ou la digoxigénine.

Un rapporteur d'une enzyme est par exemple le substrat de la dite enzyme.

Un rapporteur d'une molécule permettant de produire un composé luminescent est par exemple un substrat, une enzyme ou un catalyseur.

Un rapporteur de la biotine est par exemple l'avidine, la streptavidine ou la neutravidine, de préférence couplée à un marqueur direct ou à un marqueur indirect, tel qu'une enzyme.

Des marqueurs indirects préférés selon l'invention sont la biotine et une enzyme, de préférence une enzyme produisant un composé luminescent par réaction avec un substrat.

Un exemple d'enzyme est une peroxydase, par exemple la peroxydase de raifort (HRP), une luciférase ou une phosphatase alcaline.

Un rapporteur de la biotine préféré selon l'invention est la streptavidine couplée à une peroxydase, de préférence la peroxydase de raifort.

A titre d'exemple, si le rapporteur (appelé également premier rapporteur) du marqueur de détection indirect couplé à un ligand de détection d'un analyte est couplé à une enzyme peroxydase, il est nécessaire d'ajouter dans une étape ultérieure le rapporteur (appelé également deuxième rapporteur) de cette enzyme peroxydase, c'est-à-dire un substrat de cette enzyme, tel que le luminol, l'isoluminol et/ou un dérivé du luminol ou de l'isoluminol. Dans ce cas, le deuxième rapporteur est un substrat.

### Support solide

Le ou les supports utilisés pour la mise en œuvre d'un procédé d'analyse selon l'invention sont des supports solides.

Un support solide peut être en toute matière appropriée pour la mise en œuvre d'un procédé d'analyse.

Un support solide est par exemple un support à base d'un polymère ou d'un mélange de polymères. Un support solide approprié selon l'invention est, par exemple, un support en polystyrène, polypropylène, poly(méth)acrylate, polybutadiène ou leurs combinaisons.

Un support solide préféré est en polystyrène et/ou polypropylène.

Un autre type de support solide approprié selon l'invention est par exemple un support solide inorganique, tel que le verre.

Un support peut par exemple être sous la forme d'une plaque ou d'une microplaque.

Un support solide comprend au moins deux compartiments, qui sont également appelés zones d'analyse. Les compartiments d'un support solide définissent l'orientation du support solide. Le dessus d'un support solide (appelé également face supérieure du support solide) se situe du côté du ou des compartiments et donc du côté du ou des spots. Le dessous d'un support solide (appelé également face inférieure d'un support solide) est la face opposée.

Selon l'invention, un support solide, qui peut être par exemple une microplaque, comprend au moins deux compartiments.

Lorsqu'un support solide comprend au moins deux compartiments, ils sont isolés les uns des autres, de sorte qu'ils ne communiquent pas entre eux, c'est-à-dire de sorte que les fluides (appelées également solutions) utilisés lors de la mise en œuvre d'un procédé d'analyse ne puissent pas circuler d'un compartiment à un autre pendant le procédé d'analyse.

Ainsi, une solution ajoutée dans un compartiment ne va pas dans les autres compartiments. Par exemple, le ou les compartiments comprennent ou sont constitués d'un fond et une ou plusieurs parois, la ou lesdites parois isolant le ou les compartiments les uns des autres de sorte qu'ils ne communiquent pas entre eux.

Un support solide est, de préférence, une microplaque. Dans ce cas, un exemple de compartiment est un puits. La microplaque est typiquement une microplaque de 96 puits ou de 384 puits.

Dans un mode de réalisation particulier, lorsqu'un support solide comprend au moins deux compartiments, ils peuvent en outre être isolés les uns des autres, de sorte qu'un signal émis au niveau d'un compartiment ne soit pas, en partie ou en totalité, détecté dans un autre compartiment. A cet effet, la ou les parois du ou des compartiments peuvent comprendre ou être constituées d'un matériau opaque.

Par « matériau opaque », on entend notamment un matériau ne laissant pas ou ne laissant substantiellement pas passer le signal à détecter correspondant à la présence d'un analyte. Par « ne laissant substantiellement pas passer le signal à détecter », on entend que le matériau opaque laisse passer au plus 20%, de préférence au plus 15%, plus préférentiellement au plus 10%, plus préférentiellement encore au plus 5%, et plus préférentiellement encore au plus 2%, au plus 1% ou au plus 0,5% du signal à détecter. Un exemple de matériau opaque est un matériau noir.

Dans un autre mode de réalisation particulier, lorsqu'un support solide comprend au moins deux compartiments, la ou les parois du ou des compartiments comprennent ou sont constituées d'un matériau transparent.

Dans un autre mode de réalisation particulier, lorsqu'un support solide comprend au moins deux compartiments, le, les ou des compartiments peuvent comprendre au moins une paroi constituée d'un matériau transparent et au moins une paroi constituée d'un matériau opaque.

Par « matériau transparent », on entend notamment un matériau laissant passer au moins 80% d'un signal à détecter correspondant à la présence d'un analyte, de préférence au moins 85% du signal à détecter, plus préférentiellement au moins 90% du signal à détecter, plus préférentiellement au moins 95% du signal à détecter.

Dans un mode de réalisation préféré, le fond du ou des compartiments d'un support solide comprend ou est constitué d'un matériau transparent, afin de permettre la détection du signal à détecter correspondant à la présence d'un analyte par le fond du compartiment.

Des exemples de matériau opaque sont le verre coloré, polystyrène coloré, polyéthylène coloré, polypropylène coloré et leurs combinaisons.

Des exemples de matériau transparent sont le verre, polystyrène, polyméthylpentène, polycarbonate, acrylonitrile butadiène styrène, polyméthacrylate de méthyle ou leurs combinaisons.

Typiquement, au moins un (par exemple un ou deux) compartiment d'un support solide est utilisé par échantillon à analyser.

Un compartiment d'un support solide utilisé pour analyser un échantillon comprend au moins deux spots, au moins trois spots, par exemple trois spots, quatre spots ou cinq spots, ou au moins six spots, de préférence six spots, sept spots, huit spots, plus préférentiellement au moins neuf spots, par exemple neuf spots, dix spots, onze spots, douze spots, treize spots, quatorze spots, quinze spots, seize spots ou plus de seize spots.

Par « spot », on entend ici une zone située à la surface du fond d'un compartiment d'un support solide comprenant au moins un composé d'intérêt. Le ou les composés d'intérêt peuvent être fixés à la surface du fond d'un compartiment par des interactions physico-chimiques non covalentes (par exemple de type liaisons faibles, et en particulier ioniques, van der Waals, hydrogène et/ou hydrophobe) et/ou par des liaisons covalentes.

Un spot peut comprendre, outre le ou les composé(s) d'intérêt, au moins un polymère, en particulier au moins un polymère comportant des groupes hydrophiles, par exemple au moins un hydrogel.

Par « au moins », on entend dans la présente demande un ou plusieurs, « plusieurs » signifiant en particulier deux, trois, quatre, cinq, six, sept, huit, neuf, dix, onze, douze, treize, quatorze, quinze, seize ou plus de seize.

Un spot correspond à une zone bien délimitée, généralement de petite dimension, par exemple comprise de 0,0078 mm² à 5,309 mm², de préférence de 0,196 mm² à 3,142 mm², plus préférentiellement comprise de 0,503 mm² à 2,011 mm².

Un spot peut être de forme discoïdale ou approximativement de forme discoïdale, par exemple ovale, en particulier lorsqu'un support solide est une microplaque ou une lame.

Alternativement, un spot peut être de forme carrée ou rectangulaire (en particulier une bande), par exemple lorsqu'un support solide est une membrane, ou de tout autre forme.

Les spots sont obtenus par des techniques bien connues de l'homme du métier (voir par exemple les documents US 7 470 547, US 6 576 295, US 5 916 524 ou US 5 743 960).

Par exemple, un spot est obtenu par le dépôt d'au moins une goutte d'une solution contenant une quantité déterminée dudit ou desdits composé(s) d'intérêt à un endroit précis à la surface du compartiment.

Lorsqu'un spot comprend au moins un polymère (par exemple au moins un hydrogel), ledit spot peut être obtenu par le dépôt d'au moins une goutte d'une solution contenant une quantité déterminée dudit ou desdits composé(s) d'intérêt à un endroit précis à la surface du compartiment sur laquelle ledit polymère a été déposé au préalable.

Un spot peut également être obtenu par synthèse *in situ* dudit ou desdits composé(s) d'intérêt à un endroit précis à la surface du compartiment. Ledit ou lesdits composés d'intérêt sont dans ce cas qualifiés de sonde. Il peut s'agir d'un acide nucléique ou d'un peptide (voir par exemple le document US 5 143 854).

Le composé d'intérêt peut être, par exemple, un ligand de capture, une molécule porteuse couplée à un marqueur indirect, un marqueur indirect ou un fluorophore.

Dans un mode de réalisation préféré, au moins un spot d'un compartiment comprend au moins un ligand de capture spécifique d'un analyte à détecter.

Dans un mode de réalisation avantageux, au moins un spot d'un compartiment, de préférence tous les spots d'un compartiment, comprennent au moins deux composés d'intérêt, l'un de ces composés d'intérêt étant un fluorophore. Ledit fluorophore est alors notamment utilisé pour contrôler la présence, la localisation et/ou l'intégrité des spots à l'issue d'un procédé d'analyse, en particulier d'un procédé d'analyse multiplexe. Par exemple, au moins un spot d'un compartiment comprend au moins un ligand de détection spécifique d'un analyte et au moins un fluorophore.

Dans un mode de réalisation avantageux, chaque compartiment d'un support solide comprend le même nombre de spots. De plus, chaque compartiment d'un support solide peut comprendre le même nombre de spots et la même composition en spots.

Dans un autre mode de réalisation avantageux, un support peut comprendre un ou plusieurs compartiments sans spot, ou encore avec un nombre et/ou une composition de spots différent(s). Un support solide peut par exemple comprendre au moins deux groupes distincts (ou types) de compartiments, chacun des groupes distincts ayant un nombre et/ou une composition de spots différent(s).

Un compartiment comprend généralement au moins un spot par analyte à détecter, chaque analyte pouvant par exemple correspondre à une infection ou une maladie à détecter, à l'évolution d'une infection ou maladie, à une condition (pathologique ou non) d'un sujet, à un risque de développer une condition (pathologique ou non) ou encore à un marqueur de résistance à un traitement. Plusieurs spots d'un compartiment peuvent également être destinés à l'analyse d'un même analyte.

Un compartiment comprend donc au moins un spot destiné à la détection d'un analyte, de préférence au moins deux spots destinés à la détection d'un analyte.

Un même spot peut comprendre plusieurs ligands de capture différents (par exemple plusieurs anticorps et/ou antigènes), qui sont par exemple spécifiques d'une même infection ou maladie à détecter (en particulier spécifiques d'un même virus, d'une même bactérie ou d'un même parasite), ou spécifiques d'une même évolution d'une infection ou maladie, d'une même condition (pathologique ou non) d'un sujet, d'un même risque de développer une condition (pathologique ou non) ou d'un même marqueur de résistance à un traitement.

Dans un mode de réalisation avantageux, un compartiment comprend au moins un spot contrôle, permettant de valider au moins une étape d'un procédé d'analyse, en particulier d'un procédé d'analyse multiplexe.

### Détection du signal

La détection du signal dépend du type de marqueur utilisé.

Le signal détecté est un rayonnement électromagnétique.

Le rayonnement électromagnétique peut être de la lumière, par exemple un rayonnement ultra-violet, de la lumière visible ou un rayonnement infrarouge. Le rayonnement ultra-violet est un rayonnement électromagnétique d'une longueur d'onde 10 à 380 nm. La lumière visible est un rayonnement électromagnétique d'une longueur d'onde comprise de 380 nm à 780 nm. Un rayonnement infrarouge est un rayonnement électromagnétique d'une longueur d'onde comprise de 780 nm à 1 mm.

Les expressions « détection du signal » et « acquisition du signal » sont ici synonymes.

Par « détection du signal », on entend notamment la détection d'un signal correspondant à la présence d'un analyte ou la détection d'un signal correspondant à un contrôle du procédé.

L'homme du métier sait comment détecter un signal au niveau d'un spot en fonction du ou des marqueurs de détection utilisés. Le signal est par exemple détecté au moyen d'une caméra qui capture l'image du dessous du support solide.

La détection du signal comprend généralement une mesure de l'intensité du signal, par exemple exprimée en RLU (*Relative Light Unit*).

Le signal émis par un marqueur direct de type fluorophore peut être lu directement en fluorescence, après excitation par une énergie lumineuse.

En effet, un fluorophore, appelé également fluorochrome ou molécule fluorescente, est une substance chimique capable d'émettre de la lumière de fluorescence après excitation avec une énergie lumineuse.

Comme indiqué précédemment, un marqueur indirect, par exemple de type enzyme ou de type biotine, nécessite l'ajout d'un rapporteur, le rapporteur pouvant être couplé à un marqueur direct ou indirect. Si un rapporteur est lui-même couplé à un marqueur indirect, par exemple une enzyme, il est nécessaire d'ajouter dans une étape ultérieure un rapporteur de ce marqueur indirect, par exemple le substrat de cette enzyme.

Dans le cadre de la présente invention, le ou les colorants utilisés doivent être présents lors de la détection du signal et la détection du signal est réalisée en présence d'une phase liquide.

En utilisant un colorant selon l'invention, il est possible de diminuer le bruit de fond et notamment d'améliorer le rapport « signal détecté sur bruit de fond », en détectant le signal correspondant à la présence d'un analyte émis au niveau des spots par le dessous du support solide.

Dans un mode de réalisation préféré, le signal détecté dans le procédé d'analyse selon l'invention est un signal émis en chimiluminescence par un composé chimiluminescent.

La chimiluminescence est une réaction chimique ayant pour conséquence la production de lumière. Une réaction de ce type est l'oxydo-réduction du luminol (3-aminophthalhydrazide, appelé également 5-amino-2,3-dihydro-phthalazine-1,4-dione, de formule brute C₈H₇N₃O₂), de l'isoluminol ou d'un dérivé du luminol ou de l'isoluminol par un oxydant, par exemple l'eau oxygénée ou un hydroxyde quelconque. Durant une réaction de chimiluminescence, la molécule produite par la réaction se trouve dans un état excité : il s'agit du composé chimiluminescent. C'est le retour de ce composé chimiluminescent à l'état fondamental qui provoque l'émission de lumière.

Dans un mode de réalisation préféré, le signal détecté en chimiluminescence est émis par la réaction d'une enzyme peroxydase avec son substrat, par exemple le luminol, l'isoluminol (appelé également 4-aminophthalhydrazide) et/ou un dérivé du luminol ou de l'isoluminol. Cette réaction nécessite également la présence d'un oxydant et, le cas échéant, d'un médiateur d'électrons.

Un dérivé du luminol ou de l'isoluminol est, de préférence, une molécule obtenue à partir du luminol ou de l'isoluminol par toute(s) modification(s) possible(s) (par exemple chimique et/ou enzymatique). Un dérivé du luminol ou de l'isoluminol est en particulier un substrat d'une enzyme peroxydase, la réaction de ladite enzyme peroxydase avec ledit dérivé du luminol ou isoluminol permettant la production d'un composé chimioluminescent.

Un dérivé de l'isoluminol peut être par exemple l'aminoethylisoluminol (ou AEI), l'aminoethylethylisoluminol (ou AEEI), l'aminobutylisoluminol (ou ABI), l'aminobutyléthylisoluminol (ou ABEI), l'aminopentyléthylisoluminol (ou APEI), l'aminohexylisoluminol (ou AHI), l'aminohexyléthylisoluminol (ou AHEI), l'aminooctylmethylisoluminol (ou AOMI) ou l'aminooctylethylisoluminol (ou AOEI), tels que décrits dans la publication Dodeigne C. et al (2000), Talanta 51, 415-439, « Chemiluminescence as diagnostic tool. A review »*.*

Selon un autre mode de réalisation particulier, le signal détecté en chimiluminescence est émis par une réaction enzymatique ou chimique avec un substrat choisi parmi un composé acridine, coelenterazine, dioxetane ou peroxyoxalic, ou un de leurs dérivés, et en particulier un composé décrit dans la publication Dodeigne C. et al (2000), Talanta 51, 415-439, « Chemiluminescence as diagnostic tool. A review »*.*

Un médiateur d'électrons est, par exemple, le sodium 3-(10'phénothiazinyl)propane 1-sulfonate, le p-iodophénol, l'acide p-iodophénylboronique, l'acide 4-(phénothiazine-10-yl)butane-1-sulfonique, ou leurs combinaisons.

Un oxydant est, par exemple, un peroxyde, par exemple un peroxyde d'hydrogène, ou du perborate de sodium.

Le signal issu de la réaction d'une enzyme peroxydase avec le luminol, l'isoluminol et/ou un dérivé du luminol ou de l'isoluminol est lu à une longueur d'onde comprise de 375 nm à 580 nm, par exemple 425 nm.

Le signal détecté est, de préférence, exprimé en RLU (*Relative Light Unit*). L'enzyme peroxydase peut être couplée à un ligand de détection, par exemple à un ligand de détection spécifique d'un analyte, ou à un rapporteur d'un marqueur de détection indirect, tel que la streptavidine.

Généralement, la réaction de chimiluminescence est effectuée au moyen d'un kit comprenant au moins deux solutions.

La première solution comprend le substrat de la peroxydase, par exemple le luminol, l'isoluminol et/ou un dérivé du luminol ou de l'isoluminol, et un médiateur d'électrons ; la deuxième solution comprend un oxydant. A titre d'exemple, il est possible d'utiliser le kit « Immun-star western C » (Bio-Rad, Etats-Unis), « ELISTAR ETA C Ultra ELISA » (Cyanagen, Italie), « Supersignal West Pico » (Thermo Scientific, Etats-Unis), « Chemiluminescent Sensitive Plus HRP» (Surmodics, Etats-Unis).

### Fluorophore utilisé comme contrôle

Dans un mode de réalisation avantageux, le ou les spots d'au moins un compartiment d'un support solide comprennent un fluorophore utilisé comme contrôle.

Le fluorophore utilisé comme contrôle n'interfère, de préférence, pas ou peu avec le signal correspondant à la présence d'un analyte, par exemple avec le signal émis par un composé chimiluminescent.

A titre d'exemple, lorsque le signal correspondant à la présence d'un analyte est un composé chimiluminescent obtenu à partir du luminol, de l'isoluminol et/ou d'un dérivé du luminol ou de l'isoluminol, le fluorophore utilisé comme contrôle n'émet de préférence pas de lumière aux environs de 425 nm, en particulier de 400 nm à 550 nm, de préférence de 375 nm à 550 nm, plus préférentiellement de 350 nm à 580 nm. Il peut, par exemple, émettre de la lumière uniquement à des longueurs d'ondes inférieures (ou inférieures ou égales) à 400 nm, 390 nm, 380 nm, 375 nm, 370 nm, 360 nm ou 350 nm, ou uniquement à des longueurs d'ondes supérieures (ou supérieures ou égales) à 550 nm, 560 nm, 570 nm, 580 nm, 590 nm ou 600 nm.

Un fluorophore utilisé comme contrôle est, par exemple, sélectionné dans le groupe consistant en une coumarine, une rhodamine, une carbopyronine, une oxazine, du benzopyrylium, une phycoérythrine et leurs dérivés. Ledit fluorophore est éventuellement couplé à une molécule porteuse, par exemple une protéine telle que la BSA.

Un fluorophore utilisé comme contrôle est, par exemple, sélectionné dans le groupe consistant en une coumarine, une rhodamine, une carbopyronine, une oxazine, la B-phycoérythrine, un dérivé du benzopyrylium et leurs dérivés.

Un fluorophore utilisé comme contrôle est, de préférence, sélectionné dans le groupe consistant en une carbopyronine, un dérivé de carbopyronine, une oxazine, un dérivé d'une oxazine, un dérivé du benzopyrylium et une phycoérythrine.

Un fluorophore davantage préféré pour utilisation comme contrôle est sélectionné dans le groupe consistant en une carbopyronine, un dérivé du benzopyrylium et une phycoérythrine.

Alternativement, un fluorophore préféré pour utilisation comme contrôle peut être sélectionné dans le groupe consistant en un dérivé de carbopyronine, un dérivé du benzopyrylium et une phycoérythrine.

Un fluorophore préféré pouvant être utilisé dans les spots comme contrôle est, par exemple, une carbopyronine comprenant la structure de base suivante :

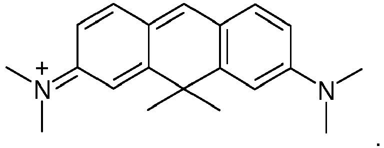

A titre d'exemple, on peut citer la carbopyronine Atto 633 commercialisée par Atto-Tec et ses dérivés, en particulier un dérivé amine de l'Atto 633.

Un autre exemple de fluorophore préféré pouvant être utilisé dans les spots comme contrôle est le fluorophore commercialisé par la société Dyomics sous le nom « Dye 634 » (sous forme couplée à une molécule porteuse, par exemple la BSA) dont la formule est la suivante :

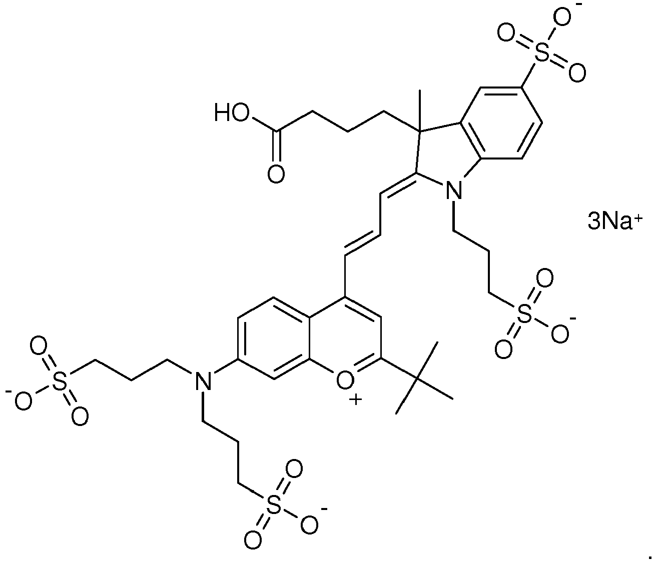

Il s'agit d'un dérivé du benzopyrylium.

Le Dye 634 peut également être utilisé sous sa forme amine (Dye 634-amine) dans les spots comme contrôle. Il peut alors être utilisé couplé ou non à une molécule porteuse et en particulier à la BSA.

Encore un autre exemple de fluorophore préféré dérivé du benzopyrylium pouvant être utilisé dans les spots comme contrôle est le dérivé amine du Dye 630, le Dye 630 ayant la formule suivante :

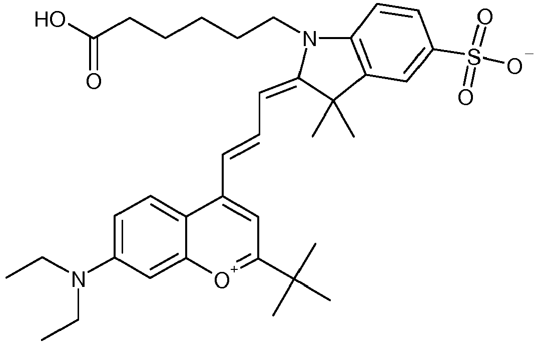

Le dérivé amine du Dye 630 peut être utilisé couplé ou non couplé à une molécule porteuse et en particulier à la BSA.

### Colorant

Par « colorant », on désigne ici un composé soluble qui absorbe la lumière dans une gamme de longueurs d'onde recouvrant partiellement ou en totalité la gamme de longueur d'onde d'émission du signal que l'on cherche à réduire (partiellement ou en totalité), i.e. du signal à l'origine des interférences lumineuses.

Lorsqu'un composé luminescent, par exemple un composé chimiluminescent, est utilisé dans un procédé d'analyse pour détecter la présence d'un analyte, un colorant selon l'invention absorbe, de préférence, la lumière dans une gamme de longueurs d'onde recouvrant en totalité la gamme de longueur d'onde d'émission dudit composé luminescent.

Typiquement, pour un système de révélation utilisant la réaction d'une peroxydase sur le luminol, l'isoluminol et/ou un dérivé du luminol ou de l'isoluminol, le colorant selon l'invention absorbe, de préférence, une gamme de longueur d'onde comprise de 375 nm à 580 nm.

Lorsqu'un fluorophore est utilisé dans un procédé d'analyse pour détecter la présence d'un analyte dans un échantillon et, éventuellement, quantifier un analyte dans un échantillon, le colorant utilisé est compatible avec la détection du signal émis par ledit fluorophore. Ainsi, le colorant utilisé n'absorbe pas la lumière dans une gamme de longueurs d'onde correspondant à la gamme de longueurs d'onde d'excitation et/ou d'émission dudit fluorophore et, de préférence, ne diffuse pas de lumière correspondant à la gamme de longueur d'onde d'excitation et/ou d'émission dudit fluorophore.

Dans un mode de réalisation préféré, le ou les colorants ne sont pas fluorescents dans le rouge, c'est-à-dire qu'ils n'émettent pas de lumière à des longueurs d'onde comprises de 620 à 780 nm lorsqu'ils sont excités par une quelconque lumière, en particulier en milieu basique.

Par exemple, un colorant préféré selon l'invention n'absorbe pas à 657 nm et, en particulier, n'absorbe pas entre 620 et 800 nm, entre 610 et 850 nm ou entre 600 et 900 nm ou entre 580 et 950 nm, et n'est lui-même pas fluorescent.

De manière surprenante, le colorant selon l'invention absorbe le signal à l'origine des interférences lumineuses, sans interférer ou en interférant peu avec le signal à détecter, par exemple en interférant pas ou peu avec la lumière émise par le composé chimiluminescent issu de la réaction d'une enzyme peroxydase avec le luminol, l'isoluminol et/ou un dérivé du luminol ou de l'isoluminol localement au niveau des spots.

L'expression « le composé X n'interfère pas ou interfère peu avec un signal » signifie ici que l'intensité du signal en présence du composé X est diminuée au plus de 20%, de préférence au plus de 15%, plus préférentiellement encore au plus de 10%, par rapport à l'intensité mesurée en l'absence dudit ou desdits colorants.

Par ailleurs, le ou les colorants selon l'invention n'induisent pas de transfert d'énergie avec le composé chimiluminescent, comme cela peut être observé avec la fluorescéine qui peut passer dans un état activé et émettre à une longueur d'onde supérieure à la longueur d'onde d'émission du composé chimiluminescent produit par la réaction d'une enzyme peroxydase avec son substrat (par exemple le luminol, l'isoluminol et/ou un dérivé du luminol ou de l'isoluminol).

Dans un mode de réalisation avantageux, un colorant selon l'invention permet en outre la détection du signal émis par un fluorophore présent en tant que contrôle dans les spots. Un tel colorant est, par exemple, obtenu ou susceptible d'être obtenu par le procédé de sélection d'un colorant tel que défini ci-dessous au paragraphe « procédé de sélection d'un colorant permettant en outre la détection du signal émis par un fluorophore présent dans le ou les spots comme contrôle ».

Un colorant selon l'invention est, par exemple, sélectionné dans le groupe consistant en la tartrazine, jaune de quinoléine, jaune de naphtol S, curcumine, riboflavine et phosphate-5' de riboflavine.

Ainsi, de manière surprenante, le ou les colorants selon l'invention permettent donc de réduire partiellement ou totalement les interférences lumineuses provenant d'un composé chimiluminescent, sans interférer ou en interférant peu avec le signal à détecter en chimiluminescence et, le cas échéant, en permettant la détection de la fluorescence émise par un fluorophore présent dans les spots en tant que contrôle.

Les inventeurs ont montré que l'utilisation d'un seul et unique colorant, en particulier la tartrazine, permet d'améliorer (et ainsi sécuriser) la détection du signal dans un procédé d'analyse sur spot(s), en particulier d'un procédé d'analyse multiplexe, et ceci sans interférer avec le signal émis par un fluorophore éventuellement présent dans les spots.

L'homme du métier peut déterminer facilement la quantité optimale de colorant(s) à utiliser dans un procédé d'analyse sur spot(s), en particulier d'un procédé d'analyse multiplexe, pour obtenir l'amélioration de la détection du signal souhaitée, en testant par exemple plusieurs concentrations du ou des colorant(s).

### Procédé de sélection d'un colorant permettant en outre la détection du signal émis par un fluorophore présent dans le ou les spots comme contrôle

Est également décrit un procédé de sélection d'un colorant permettant en outre la détection du signal émis par un fluorophore présent dans le ou les spots d'un support solide, ledit procédé comprenant les étapes suivantes :
a) mettre une phase liquide comprenant un colorant à tester ou un mélange de colorants à tester en contact du ou des spots d'un compartiment d'un support solide, au moins un des spots dudit compartiment comprenant un fluorophore,
b) détecter un signal émis par ledit fluorophore en présence de ladite phase liquide comprenant ledit colorant à tester ou ledit mélange de colorants à tester, et
c) sélectionner un colorant ou un mélange de colorants en présence duquel le signal détecté à l'étape b) permet de localiser le ou les spots comprenant ledit fluorophore.

Le colorant à tester ou compris dans le mélange à tester est un composé soluble qui absorbe la lumière dans une gamme de longueurs d'onde recouvrant partiellement ou en totalité la gamme de longueur d'onde d'émission du signal que l'on cherche à réduire (partiellement ou en totalité), i.e. du signal à l'origine des interférences lumineuses.

L'étape a) est réalisée pour chaque colorant ou mélange de colorant à tester.

Dans l'étape a), un même colorant à tester ou mélange de colorants à tester peut être ajouté dans plusieurs compartiments, par exemple à des concentrations différentes, afin de tester différentes concentrations dudit colorant ou dudit mélange de colorants.

Un compartiment différent est utilisé pour chaque colorant à tester ou mélange de colorants à tester différent.

### Composition absorbante à base d'au moins un colorant

Dans un mode de réalisation avantageux, le colorant tel que défini ci-dessus au paragraphe « colorant » est apporté sous la forme d'une composition absorbante.

Une composition absorbante est une solution aqueuse comprenant au moins un colorant.

Une composition absorbante préférée ne comprend qu'un seul colorant. Par exemple, une composition absorbante préférée comprend de la tartrazine comme seul colorant.

La composition absorbante peut comprendre de 100 µg/ml à 3000 µg/ml de colorant, de préférence 200 µg/ml à 2500 µg/ml de colorant, plus préférentiellement de 250 µg/ml à 2000 µg/ml de colorant, par exemple de 250 µg/ml à 500 µg/ml ou de 1000 µg/ml à 2000 µg/ml. Par exemple, la composition absorbante peut comprendre 250 µg/ml, 500 µg/ml, 1000 µg/ml ou 2000 µg/ml de colorant.

La composition absorbante peut comprendre de 0,187 mM à 5,614 mM de colorant, de préférence 0,374 mM à 4,678 mM de colorant, plus préférentiellement de 0,468 mM à 3,743 mM de colorant, par exemple de 0,468 mM à 0,936 mM ou de 1,871 mM à 3,743 mM. Par exemple, la composition absorbante peut comprendre 0,468 mM, 0,936 mM, 1,871 mM ou 3,743 mM de colorant.

De manière avantageuse, la composition absorbante comprend un colorant ou au moins un colorant et au moins un composé impliqué dans la production d'un composé chimiluminescent. Une telle composition absorbante présente l'avantage d'être stable au cours du temps.

L'expression « stable au cours du temps » signifie que, lors de la mise en œuvre d'un même procédé d'analyse, le signal détecté en utilisant une composition absorbante à J0 est sensiblement identique au signal détecté en utilisant ladite composition absorbante après conservation de ladite composition absorbante pendant au moins un mois à 4°C et/ou à 37°C, de préférence pendant au moins 3 mois à 4°C, par exemple pendant 3 mois, 6 mois, un an ou deux ans à 4°C.

L'expression « sensiblement identique » signifie que le signal détecté varie au plus de 40%, de préférence au plus de 30%, plus préférentiellement au plus de 20%.

La composition absorbante peut être mélangée à une ou plusieurs compositions et/ou à un ou plusieurs composés utilisés dans le cadre d'un procédé d'analyse, en particulier utilisés au cours de la ou des étapes de révélation.

Dans un mode de réalisation avantageux, la composition absorbante comprend en outre au moins un composé sélectionné dans le groupe consistant en du luminol, l'isoluminol, un dérivé du luminol ou de l'isoluminol, un médiateur d'électrons et un oxydant.

Le luminol, l'isoluminol, le dérivé du luminol ou de l'isoluminol, l'enzyme peroxydase, le médiateur d'électrons et l'oxydant sont notamment tels que définis ci-dessus.

Une composition absorbante préférée comprend au moins un colorant, par exemple la tartrazine, au moins un composé sélectionné parmi le luminol, l'isoluminol ou un dérivé du luminol ou de l'isoluminol, et éventuellement un médiateur d'électrons.

Une autre composition absorbante préférée comprend au moins un colorant, par exemple la tartrazine, et au moins un oxydant, par exemple un peroxyde.

Les compositions absorbantes comprennent avantageusement au moins un solvant.

Un solvant utilisé dans la composition absorbante est, de préférence, compatible avec une réaction enzymatique conduisant à la production d'un composé luminescent, telle que la réaction de la peroxydase avec le luminol, l'isoluminol et/ou un dérivé du luminol ou de l'isoluminol.

Un solvant préféré pouvant être utilisé dans la composition absorbante est de l'eau.

Un autre exemple de composition absorbante préférée comprend au moins un colorant, par exemple la tartrazine, et ne comprend pas de luminol, isoluminol, dérivé du luminol ou de l'isoluminol, médiateur d'électrons, ni oxydant. Par exemple, une telle composition absorbante comprend ou consiste en au moins un colorant, par exemple un unique colorant, et un solvant, par exemple de l'eau.

### Amélioration de la détection du signal

L'utilisation d'au moins un colorant tel que défini ci-dessus ou d'une composition absorbante le comprenant telle que définie ci-dessus permet d'améliorer (et ainsi de sécuriser) la détection du signal dans un procédé d'analyse sur spot(s), de préférence un procédé d'analyse multiplexe sur spots, en particulier lorsque la détection du signal est effectuée en présence d'une phase liquide.

Par « améliorer la détection du signal », on entend notamment la diminution du bruit de fond, et plus particulièrement l'amélioration du rapport « signal détecté sur bruit de fond », en présence d'au moins un colorant ou de la composition absorbante le comprenant, par rapport au rapport « signal détecté sur bruit de fond » obtenu en leur absence.

Le « signal détecté » pour évaluer l'amélioration du rapport « signal détecté sur bruit de fond » est, par exemple, l'intensité du signal mesurée au niveau d'un spot donné (i.e. à l'endroit où se situe ledit spot) en présence d'un analyte à détecter dans un échantillon ou mesurée au niveau d'un spot donné (i.e. à l'endroit où se situe ledit spot) en présence d'une quantité connue d'un analyte à détecter.

De préférence, le « signal détecté » pour évaluer l'amélioration du rapport « signal détecté sur bruit de fond » est l'intensité du signal mesurée au niveau d'un spot donné (i.e. à l'endroit où se situe ledit spot) en présence d'un analyte présent dans une quantité qui induit, en l'absence d'un colorant, des interférences lumineuses, telles qu'un arc lumineux, un spot jumeau et/ou un voile lumineux.

L'intensité du signal mesurée au niveau d'un spot est généralement exprimée en RLU (*Relative Light Unit*).

L'homme du métier sait comment détecter le signal au niveau d'un spot (i.e. à l'endroit où se situe ledit spot), en fonction du ou des marqueurs de détection utilisés, en particulier au moyen d'une caméra qui est avantageusement située en dessous du support solide.

Le « bruit de fond » est l'intensité de lumière mesurée au niveau des zones du fond d'un compartiment d'un support solide qui ne comprennent pas de spots.

Le bruit de fond est généralement exprimé en RLU (*Relative Light Unit*).

Une amélioration du rapport « signal détecté sur bruit de fond » est présente lorsque le rapport « signal détecté sur bruit de fond » est augmenté, par exemple en augmentant le signal détecté et en diminuant le bruit de fond, ou en diminuant le signal détecté et en diminuant encore plus fortement le bruit de fond.

L'utilisation d'un colorant selon l'invention permet, de préférence, une augmentation du rapport « signal détecté sur bruit de fond », en présence du ou des colorants versus en l'absence du ou des colorants, d'au moins 5%, de préférence d'au moins 10%, plus préférentiellement d'au moins 15%, plus préférentiellement d'au moins 20%, plus préférentiellement encore d'au moins 25%, par exemple d'au moins 30%, ou d'au moins 40%.

La présente invention est particulièrement adaptée à un procédé d'analyse sur spot(s), en particulier d'un procédé d'analyse multiplexe sur spots, basé sur une révélation en chimiluminescence. En effet, afin que le signal émis en chimiluminescence permette une détection de l'interaction ligand de capture-analyte-ligand de détection par une amplification du signal, il est nécessaire que la réaction enzymatique se poursuive lors de la détection du signal, et donc que le substrat de l'enzyme soit présent dans une phase liquide au niveau des spots, au moment de la détection du signal.

Dans le cadre de la présente invention, le colorant, par exemple apporté sous la forme d'une composition absorbante, doit être présent au moment de la détection du signal.

Le colorant peut être ajouté avant, en même temps ou ultérieurement à l'ajout d'un ou plusieurs des composés nécessaires à la réaction de chimiluminescence. Dans tous les cas, le colorant doit être présent au moment de l'acquisition du signal.

Les composés nécessaires à une réaction de chimiluminescence sont généralement une enzyme (par exemple une enzyme peroxydase), un substrat de l'enzyme (par exemple le luminol, l'isoluminol et/ou un dérivé du luminol ou de l'isoluminol), éventuellement au moins un autre composé tel qu'un oxydant (par exemple un peroxyde) et/ou un médiateur d'électrons (par exemple, le sodium 3-(10'phénothiazinyl)propane 1-sulfonate).

### Procédé pour améliorer la détection d'un signal

La présente invention a particulièrement pour objet un procédé d'analyse multiplexe, permettant d'améliorer (et ainsi de sécuriser) la détection d'un signal correspondant à la présence d'un analyte, ledit procédé comprenant ou consistant en les étapes suivantes :
a) fournir un support solide comprenant au moins deux compartiments, lesdits compartiments comprenant au moins deux spots destinés à la détection d'un analyte,
b) mettre un échantillon à analyser en présence des spots d'un compartiment,
c) mettre en présence des spots dudit compartiment au moins un ligand de détection d'un analyte, ledit ligand de détection d'un analyte étant couplé à un marqueur de détection direct ou indirect,
d) lorsque ledit marqueur de détection est un marqueur indirect, mettre en présence des spots dudit compartiment un rapporteur du marqueur de détection indirect couplé audit ligand de détection,
e) lorsque le rapporteur utilisé à l'étape d) est couplé à un marqueur indirect, mettre en présence des spots dudit compartiment un rapporteur du marqueur de détection indirect couplé audit rapporteur,
f) mettre au moins un colorant en présence des spots dudit compartiment, ledit ou lesdits colorants étant compris dans une phase liquide en contact des spots dudit compartiment, et ledit au moins un colorant étant la tartrazine, et
g) détecter un signal correspondant à la présence d'un analyte au niveau des spots dudit compartiment, en présence d'une phase liquide comprenant ledit ou lesdits colorants,
ledit procédé comprenant en outre une ou plusieurs étapes de lavage permettant d'éliminer les composés non liés aux spots ou aux différents composés liés directement ou indirectement aux spots,
aucune étape de lavage n'étant effectuée entre l'étape f) et l'étape g).

Le procédé selon l'invention comprend, de préférence, d'abord l'étape a) ; puis les étapes b) et c) qui peuvent être réalisées dans cet ordre, ou l'étape c) avant l'étape b), ou encore les étapes b) et c) simultanément ; puis l'étape d) ; puis les étapes e) et f) qui peuvent être réalisées dans cet ordre, ou l'étape f) avant l'étape e), ou encore les étapes e) et f) simultanément ; puis l'étape g).

Dans un mode de réalisation préféré, l'étape f) est réalisée simultanément à l'étape e).

Aucune étape de lavage n'est effectuée entre l'étape f) et l'étape g) (que l'étape e) soit réalisée avant, après, ou simultanément à l'étape f)), de sorte que le colorant soit présent au moment de la détection du signal.

Lorsque l'étape c) est réalisée avant l'étape b), il n'y a pas d'étape de lavage entre l'étape c) et l'étape b).

L'expression « mettre un composé X en présence d'un ou des spots d'un compartiment » signifie notamment que le composé X est ajouté dans un compartiment comprenant ledit ou lesdits spots, ledit compartiment étant de préférence destiné à analyser un échantillon, et ledit composé X étant de préférence apporté sous la forme d'une composition le comprenant, telle qu'une solution, une dispersion ou une suspension.

Lorsqu'au moins deux composés sont à mettre en présence du ou des spots d'un compartiment au cours d'une même étape et/ou lorsqu'au moins deux des étapes b) à f) sont réalisées simultanément, lesdits composés peuvent être mis en présence dudit ou desdits spots séparément, c'est-à-dire apportés sous la forme de compositions distinctes (en particulier sous la forme de solutions, dispersions ou suspensions distinctes) ; alternativement, lesdits composés ou certains desdits composés peuvent être mis en présence du ou des spots d'un compartiment sous la forme d'un ou plusieurs mélanges.

Les différents composés sont mis en présence des spots d'au moins un compartiment pendant une certaine durée, par exemple de 1 seconde à 2 heures, de préférence 1 minute à 1 heure, plus préférentiellement 5 minutes à 50 minutes, plus préférentiellement encore de 10 minutes à 40 minutes.

L'homme du métier sait déterminer la température appropriée pour chaque étape d'incubation. La température d'une incubation peut par exemple être de 4°C, une température comprise de 19°C à 24°C, 37°C ou 40°C.

Les différents constituants utilisés au cours des étapes b), c), d) et e) sont bien connus de l'homme du métier. Ils permettent par exemple la formation de complexes antigènes-anticorps et marqueur-rapporteur.

Le procédé comprend en outre une ou plusieurs étapes de lavage qui permettent d'éliminer les composés non liés aux spots ou aux différents composés liés directement ou indirectement aux spots.

Typiquement, une étape de lavage consiste en au moins un cycle, de préférence au moins deux cycles, plus préférentiellement 3 à 6 cycles, de distribution (par exemple un volume de 400 µl) et aspiration d'une solution de lavage dans chaque compartiment utilisé.

Les étapes b) à g) sont notamment effectuées pour chaque compartiment d'un support solide comprenant au moins un spot destiné à la détection d'un analyte, dans lequel un échantillon est analysé.

L'étape a) consiste à fournir un support solide comprenant au moins deux compartiments, lesdits compartiments comprenant au moins deux spots destinés à la détection d'un analyte.

L'étape a) signifie notamment que le procédé d'analyse est mise en œuvre au moyen dudit support solide, c'est-à-dire en utilisant ledit support solide.

Un support solide est notamment tel que défini ci-dessus au paragraphe « support solide ».

Dans un mode de réalisation avantageux, un support solide comprend au moins un compartiment dont au moins un spot comprend un fluorophore en tant que contrôle du ou des spots ; de préférence, un support solide comprend au moins un compartiment dont les spots comprennent un fluorophore en tant que contrôle des spots.

Dans l'étape b), un échantillon à analyser est mis en présence du ou des spots d'un compartiment du support solide.

L'échantillon à analyser et le ou les analytes à détecter sont notamment tels que définis ci-dessus aux paragraphes « échantillon » et « analyte ».

Dans l'étape c), au moins un ligand de détection d'un analyte est mis en présence du ou des spots dudit compartiment, ledit ligand de détection d'un analyte étant couplé à un marqueur de détection direct ou indirect.

Le ligand de détection d'un analyte est notamment tel que défini ci-dessus.

Dans un mode de réalisation préféré, un ligand de détection d'un analyte est couplé à un marqueur de détection indirect sélectionné dans le groupe consistant en biotine, avidine, streptavidine et neutravidine.

Lorsque le marqueur de détection est un marqueur indirect, le procédé comprend une étape d) comprenant ou consistant à mettre en présence du ou des spots dudit compartiment un rapporteur (appelé également premier rapporteur) du marqueur de détection indirect couplé audit ligand de détection, ledit rapporteur étant lui-même couplé à un marqueur direct ou indirect.

Dans un mode de réalisation préféré, un rapporteur du marqueur de détection indirect couplé à un ligand de détection d'un analyte est sélectionné dans le groupe consistant en biotine, avidine, streptavidine et neutravidine.

Par exemple, un ligand de détection d'un analyte est couplé à la biotine et le rapporteur de la biotine est la streptavidine couplée à un marqueur de détection direct ou indirect.

Lorsque le rapporteur (c'est-à-dire le premier rapporteur) utilisé à l'étape d) est couplé à un marqueur indirect, le procédé comprend en outre une étape e) consistant à mettre en présence du ou des spots dudit compartiment un rapporteur (c'est-à-dire un deuxième rapporteur) du marqueur de détection indirect couplé audit rapporteur.

Par exemple, un ligand de détection d'un analyte est couplé à la biotine et le rapporteur de la biotine est la streptavidine couplée à une enzyme. Le rapporteur de l'enzyme (c'est-à-dire le deuxième rapporteur) est alors le substrat de ladite enzyme.

Dans l'étape f), au moins un colorant est mis en présence du ou des spots dudit compartiment.

Le colorant est notamment tel que défini ci-dessus au paragraphe « colorant ».

Le colorant est, par exemple, sélectionné dans le groupe consistant en la tartrazine, jaune de quinoléine, jaune de naphtol S, curcumine, riboflavine et phosphate-5' de riboflavine.

Le colorant peut être apporté sous la forme d'une composition absorbante telle que définie ci-dessus dans le paragraphe « composition absorbante ».

Dans un mode de réalisation avantageux, le colorant est apporté sous la forme d'une composition absorbante comprenant au moins un composé sélectionné dans le groupe consistant en du luminol, de l'isoluminol, un dérivé du luminol ou de l'isoluminol, un médiateur d'électrons et un oxydant.

Lorsque la composition absorbante comprend en outre au moins un composé sélectionné dans le groupe consistant en du luminol, l'isoluminol, un dérivé du luminol ou de l'isoluminol, un médiateur d'électrons et un oxydant, les étapes e) et f) sont donc réalisées simultanément.

A l'issue de l'étape f), le ou les colorants sont compris dans une phase liquide en contact du ou des spots dudit compartiment.

Par « phase liquide en contact du ou des spots dudit compartiment », on entend ici qu'une composition liquide est présente dans ledit compartiment. Ladite composition peut par exemple être une solution, une dispersion ou une suspension.

La phase liquide à l'étape f) peut comprendre ou consister en une composition absorbante selon l'invention, dépendant notamment de si l'étape f) est réalisée avant l'étape e).

Par exemple, lorsque l'étape f) est réalisée avant l'étape e) et que l'étape f) est précédée d'au moins une étape de lavage, la phase liquide à l'étape e) peut consister en une composition absorbante. Lorsque l'étape f) est réalisée simultanément ou après l'étape e), la phase liquide à l'étape e) peut comprendre la composition absorbante.

L'étape g) comprend la détection d'un signal correspondant à la présence d'un analyte au niveau du ou des spots dudit compartiment, la détection dudit signal étant effectuée en présence de la phase liquide comprenant ledit ou lesdits colorants.

La phase liquide à l'étape g) peut être identique à la phase liquide de l'étape f), notamment lorsque l'étape f) est réalisée après l'étape e).

La phase liquide à l'étape g) peut être différente de la phase liquide de l'étape f), notamment lorsque l'étape f) est réalisée avant l'étape e).

Lorsque le colorant est apporté sous la forme d'une composition absorbante, la phase liquide à l'étape g) comprend ou consiste en ladite composition absorbante.

Lorsque plusieurs marqueurs de détection différents sont utilisés dans le procédé d'analyse pour la détection du ou des analytes, l'étape g) comprend la détection d'autant de signaux différents que de marqueurs de détection utilisés.

Le signal détecté à l'étape g) correspondant à la présence d'un analyte est par exemple le signal émis par un composé luminescent, de préférence un composé chimiluminescent, et/ou le signal émis par un fluorophore.

De préférence, le signal détecté à l'étape g) correspondant à la présence d'un analyte est le signal émis par un composé chimiluminescent.

Ainsi, l'étape g) comprend au moins la détection d'un signal émis au niveau du ou des spots dudit compartiment qui correspond à la présence d'un analyte, le signal correspondant à la présence d'un analyte étant, de préférence, émis pas un composé chimiluminescent.

En outre, l'étape g) peut avantageusement comprendre la détection d'un signal émis par un fluorophore présent en tant que contrôle dans un, plusieurs ou les spots d'au moins un compartiment du support solide.

L'homme du métier sait comment mesurer le signal émis, par exemple par un composé luminescent ou par un fluorophore, en fonction de la nature dudit composé luminescent ou dudit fluorophore.

Le signal est, de préférence, détecté à l'étape g) par le dessous du support solide.

La détection du signal comprend, de préférence, une mesure de l'intensité du signal émis au niveau du ou des spots, ladite mesure étant de préférence effectuée par le dessous du support solide, c'est-à-dire au niveau de la face inférieure du support solide.

La détection du signal est notamment réalisée au moyen d'une caméra capturant l'image du dessous du support solide. Le signal mesuré est donc le signal traversant le support solide en direction de la face inférieure dudit support solide.

La caméra peut, par exemple, être dirigée vers le dessous du support solide ou encore peut capturer l'image du dessous du support solide au moyen d'un système optique (qui peut par exemple comprendre ou consister en un ou plusieurs miroirs, en un prisme et/ou en une ou plusieurs lentilles).

La mesure de l'intensité du signal émis par un fluorophore nécessite d'éclairer le ou les compartiments, de préférence par le dessous du support solide, avec une lumière correspondant au spectre d'excitation du fluorophore.

Le procédé selon l'invention tel que défini ci-dessus permet ainsi d'améliorer la détection d'un signal correspondant à la présence d'un analyte dans un procédé d'analyse sur spot(s), en particulier lorsque la détection du signal est réalisée en présence d'une phase liquide.

L'amélioration de la détection du signal comprend ou consiste en une diminution du bruit de fond, de préférence en une augmentation du rapport « signal détecté sur bruit de fond ».

Le rapport « signal détecté sur bruit de fond » est notamment tel que défini ci-dessus.

Un procédé préféré permettant d'améliorer la détection d'un signal correspondant à la présence d'un analyte dans un procédé d'analyse multiplexe, est un procédé tel que défini ci-dessus comprenant les étapes suivantes :
a) fournir un support solide comprenant au moins deux compartiments, lesdits compartiments comprenant au moins deux spots à la détection d'un analyte, lesdits spots comprenant un ligand de capture dudit analyte et, de préférence, un fluorophore,
b) mettre un échantillon à analyser en présence des spots d'un compartiment,
c) mettre en présence des spots dudit compartiment au moins un ligand de détection d'un analyte, ledit ligand de détection d'un analyte étant couplé à un marqueur de détection indirect, de préférence la biotine,
d) mettre en présence des spots dudit compartiment un rapporteur du marqueur de détection indirect couplé audit ligand de détection, de préférence la streptavidine,
e) lorsque le rapporteur utilisé à l'étape d) est couplé à une enzyme peroxydase, mettre en présence des spots dudit compartiment un substrat de ladite enzyme, par exemple du luminol, l'isoluminol et/ou un dérivé du luminol ou de l'isoluminol,
e1) lorsque le rapporteur utilisé à l'étape d) est couplé à une enzyme peroxydase, mettre en présence du ou des spots dudit compartiment au moins un antioxydant, par exemple du peroxyde, et éventuellement au moins un médiateur d'électrons, par exemple du sodium 3-(10'phénothiazinyl)propane 1-sulfonate, ladite étape e1) pouvant être réalisée avant l'étape e), après l'étape e) ou simultanément à l'étape e),
f) mettre au moins un colorant, dont au moins la tartrazine, en présence des spots dudit compartiment, ledit ou lesdits colorants étant compris dans une phase liquide en contact des spots dudit compartiment, l'étape f) pouvant être réalisée avant ou après l'étape e), avant ou après l'étape e1), ou simultanément à l'étape e) et/ou l'étape e1), et
g) détecter un signal correspondant à la présence d'un analyte au niveau des spots dudit compartiment, en présence de la phase liquide comprenant ledit ou lesdits colorants.

### Utilisation d'au moins un colorant pour améliorer la détection du signal

La présente invention a particulièrement pour objet l'utilisation d'au moins un colorant dont au moins la tartrazine pour améliorer (et ainsi de sécuriser) la détection d'un signal correspondant à la présence d'un analyte dans un procédé d'analyse multiplexe sur un support solide comprenant au moins deux spots .

Le support solide est notamment tel que défini ci-dessus au paragraphe « support solide ». Le support solide comprend notamment au moins deux compartiments, lesdits compartiments comprenant au moins deux spots destinés à la détection d'un analyte.

Le colorant est notamment tel que défini ci-dessus au paragraphe « colorant ». Le colorant est, par exemple, sélectionné dans le groupe consistant en en la tartrazine, jaune de quinoléine, jaune de naphtol S, curcumine, riboflavine et phosphate-5' de riboflavine.

La présente invention a notamment pour objet l'utilisation telle que définie ci-dessus, caractérisée en ce que le colorant est la tartrazine.

Le colorant peut être apporté sous la forme d'une composition absorbante telle que définie ci-dessus dans le paragraphe « composition absorbante ».

Dans un mode de réalisation davantage préféré, la présente invention a pour objet l'utilisation de tartrazine pour améliorer la détection d'un signal correspondant à la présence d'un analyte dans un procédé d'analyse sur spot(s), en particulier dans un procédé d'analyse multiplexe sur spots.

La présente invention a plus particulièrement pour objet l'utilisation telle que définie ci-dessus, caractérisée en ce que la détection d'un signal correspondant à la présence d'un analyte est réalisée en présence d'une phase liquide.

La détection du signal comprend de préférence une mesure de l'intensité du signal émis au niveau du ou des spots, ladite mesure étant de préférence effectuée au niveau de la face inférieure du support solide. La détection du signal est notamment réalisée au moyen d'une caméra capturant l'image du dessous du support solide. Comme indiqué ci-dessus, la caméra peut, par exemple, être dirigée vers le dessous du support solide ou encore peut capturer l'image du dessous du support solide au moyen d'un système optique (qui peut par exemple comprendre ou consister en un ou plusieurs miroirs, en un prisme et/ou en une ou plusieurs lentilles).

La présente invention a particulièrement pour objet l'utilisation telle que définie ci-dessus, caractérisée en ce que le rapport signal détecté sur bruit de fond est augmenté.

Le rapport « signal détecté sur bruit de fond » est notamment tel que défini ci-dessus.

La présente invention a de préférence pour objet l'utilisation telle que définie ci-dessus, caractérisée en ce que le signal correspondant à la présence d'un analyte est un signal émis par un composé chimiluminescent et/ou un fluorophore.

La présente invention a également pour objet l'utilisation telle que définie ci-dessus dans laquelle le procédé d'analyse est mis en œuvre au moyen d'un support solide tel que défini ci-dessus, en particulier d'un support solide comprenant au moins un compartiment dont au moins un spot comprend un fluorophore en tant que contrôle des spots, de préférence d'un support solide comprenant au moins un compartiment dont les spots comprennent un fluorophore en tant que contrôle des spots.

D'autres caractéristiques et avantages de l'invention ressortiront mieux des exemples qui suivent, donnés à titre illustratif et non limitatif. Ces exemples et figures illustrent l'invention sans en limiter la portée.

### Figures

Figure 1 : Coupe transversale schématique d'un puits d'une microplaque. Trois spots réels sont représentés sur le fond du puits qui est constitué d'un film transparent. Les flèches creuses dirigées vers le bas montrent l'émission réellement utile qui part en direction de la caméra. Les flèches pleines représentent le trajet des rayons lumineux dans le puits. Ces flèches illustrent la présence d'artefacts lumineux qui ont pour origine la diffusion de lumière dans le milieu liquide, des reflets sur la paroi du puits et au niveau de l'interface liquide/air et au niveau du ménisque.

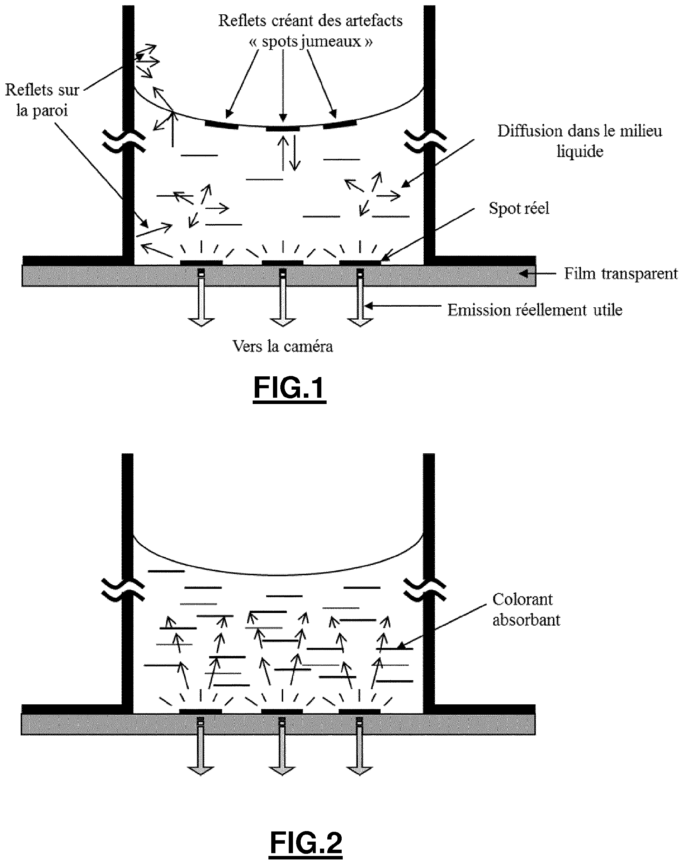

Figure 2 : Coupe transversale schématique d'un puits d'une microplaque. Trois spots réels sont représentés sur le fond du puits qui est constitué d'un film transparent. Les flèches creuses dirigées vers le bas montrent l'émission réellement utile qui part en direction de la caméra. Les flèches pleines représentent le trajet des rayons lumineux dans le puits et l'intensité de ces rayons. Les traits horizontaux représentent le colorant qui permet d'absorber la lumière émise dans le milieu liquide, diminuant ainsi la diffusion de lumière dans le milieu liquide et les artefacts lumineux au niveau des parois du puits et au niveau de l'interface liquide/air et au niveau du ménisque.
Figure 3 : Image d'un puits avec l'échantillon S1, sans ajout de tartrazine. 1 : spot jumeau lumineux de taille légèrement inférieure au spot réel, résultat du reflet du spot réel à la surface du liquide en arrière-plan. 2 : anneau lumineux. 3 : arc lumineux.
Figure 4 : Image d'un puits avec l'échantillon S1, avec 250 µg/ml de tartrazine.
Figure 5 : Image d'un puits avec l'échantillon S1, avec 500 µg/ml de tartrazine.
Figure 6 : Image d'un puits avec l'échantillon S1, avec 1000 µg/ml de tartrazine.
Figure 7 : Image d'un puits avec l'échantillon S1, avec 2000 µg/ml de tartrazine.
Figure 8 : Image d'un puits avec l'échantillon S1, avec 4000 µg/ml de tartrazine.
Figure 9 : Intensités normalisées par rapport à la condition de référence du niveau de luminosité du fond du puits (courbe inférieure) et du signal du spot de référence (courbe supérieure), en fonction de la concentration de tartrazine (µg/ml).
Figure 10 : Amélioration du rapport signal détecté sur bruit de fond en fonction de la concentration en tartrazine (µg/ml).
Figure 11 : Détection des spots en fluorescence en présence du colorant, lesdits spots comprenant un fluorophore en tant que contrôle.

### Exemples

### Matériel et Méthodes

Un procédé d'analyse multiplexe est réalisé au moyen d'une microplaque de 96 puits comprenant 9 spots par puits (3 spots numérotés de 1 à 3 sur la 1^{ère} ligne, 3 spots numérotés de 4 à 6 sur la 2^{ème} ligne et 3 spots numérotés de 7 à 9 sur la 3^{ème} ligne). Le spot numéro 9 est un spot de référence (ou spot Aref) qui comprend une molécule porteuse liée à de la biotine. Ce spot de référence permet, entre autres, de valider l'étape de révélation.

Le spot numéro 2 comprend un ligand de capture spécifique correspondant à l'analyte AH qui est présent en grande concentration dans l'échantillon de référence S1 utilisé.

Lors du procédé d'analyse, l'échantillon S1 est mis en présence des spots d'un puits pendant 40 minutes à 37°C. Après lavage du puits, un ligand de détection spécifique correspondant à l'analyte AH présent dans l'échantillon de référence S1 et couplé à la biotine est ajouté dans le puits. Après 15 minutes d'incubation à 37°C, le puits est lavé et le rapporteur streptavidine couplé à une enzyme peroxydase est ajouté dans le puits. Après 15 minutes d'incubation à 37°C, le puits est lavé. Le kit « ELISTAR ETA C Ultra ELISA » (Cyanagen, Italie) est utilisé pour l'étape de révélation, selon le manuel du fabricant. Il consiste à apporter deux solutions : une solution A qui comprend le substrat de l'enzyme, i.e. du luminol et un médiateur d'électrons (sodium 3-(10'phénothiazinyl)propane 1-sulfonate) et une solution B qui comprend un oxydant (solution de peroxyde). Avant l'acquisition du signal, une solution comprenant de la tartrazine est ajoutée, le cas échéant, dans les puits, sous la forme d'un mélange avec la solution B. Après une minute d'incubation sous agitation à 37°C, le signal est détecté.

Le signal émis en chimiluminescence par le composé chimiluminescent issu de la réaction enzymatique est mesuré au moyen d'une image prise par une caméra CCD au travers d'un objectif télécentrique par le dessous de la microplaque. Le niveau de luminosité du fond des puits est mesuré sur la même image.

Pour la mesure du signal en fluorescence, un système d'éclairage émettant une lumière rouge centrée sur la longueur d'onde de 620 nm illumine la face inférieure du support solide de manière homogène. Un filtre disposé à l'entrée de la caméra et ayant une bande passante centrée sur 680 nm permet de couper cette lumière rouge d'excitation. Il laisse passer la lumière émise par le fluorophore présent dans les spots. Le signal émis en fluorescence par les spots est mesuré à l'aide de ce dispositif.

### Résultats

### (i) Amélioration de la détection du signal en présence du colorant

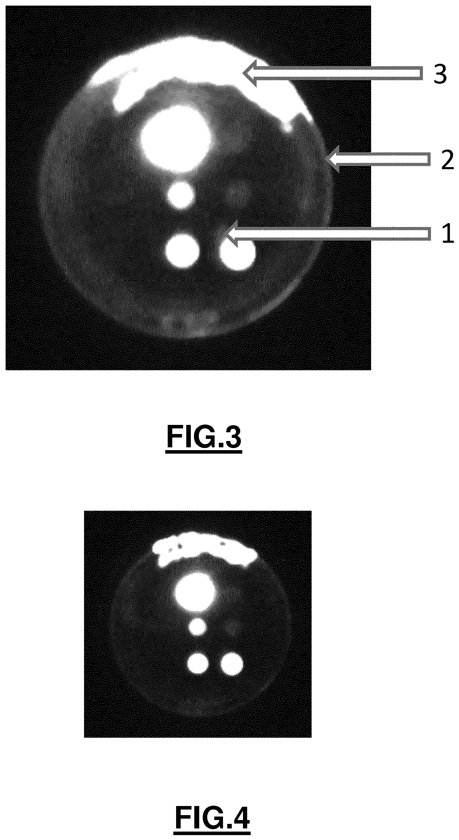

Dans le cas de l'échantillon S1, une lumière très intense est émise au niveau du spot AH (spot numéro 2 dans la grille des spots). Dans la condition de référence en l'absence de tartrazine (*cf.* *figure 3*), on observe, en décalage du spot de référence (9^{ème} spot dans la grille des spots, appelé spot Aref), un spot lumineux (1) de taille légèrement inférieure, résultat du reflet du spot de référence à la surface du liquide en arrière-plan. On observe de plus un anneau lumineux (2) sur tout le pourtour du fond du puits, résultat de l'image de la paroi verticale du puits perçue à travers le liquide jouant un rôle de lentille plan-concave. L'anneau peut s'avérer extrêmement intense près du spot AH fortement lumineux et présenter ainsi un arc lumineux (3). Un voile lumineux sur tout le fond du puits est aussi présent.

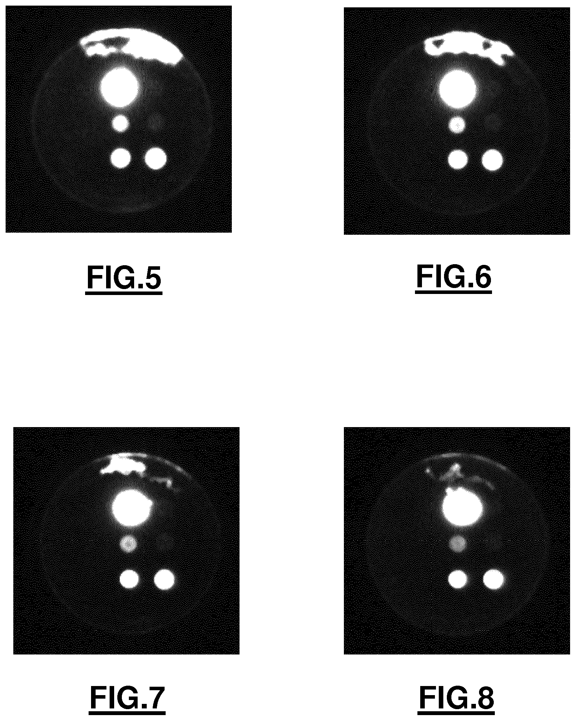

Dans les conditions avec de la tartrazine, on remarque la disparition du reflet du spot précédemment observé et la disparition de l'anneau lumineux sur le pourtour du puits, ainsi que la réduction du voile lumineux (*cf.* *figures 4 à 8*).

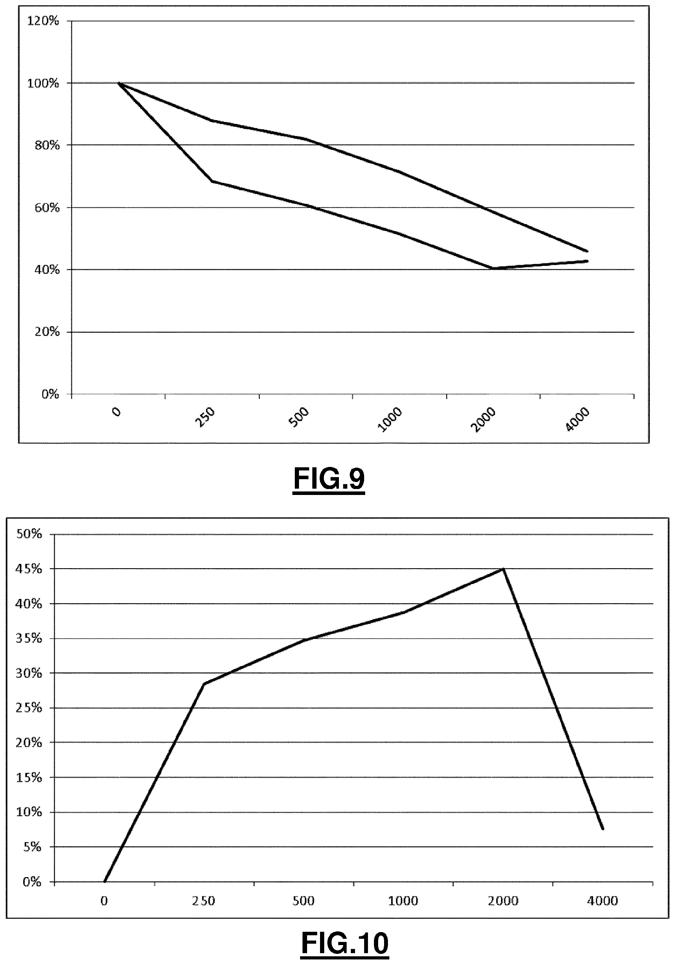

L'intensité du signal du spot de référence et le niveau de luminosité du fond du puits ont été mesurés en nombre de RLU (*Relative Light Unit*) (*cf. tableau 1 et* *figure 9*).

**Tableau 1 : Intensité du signal du spot de référence et niveau de luminosité du fond du puits**

| **Concentration en tartrazine (µg/ml)** | **Intensité du fond du puits (RLU)** | **Intensité du spot de référence (RLU)** | **Intensité du fond (%)** | **Intensité du spot de référence (%)** |
|---|---|---|---|---|
| 0 | 77 | 3168 | 100,0% | 100,0% |
| 250 | 53 | 2784 | 68,8% | 87,9% |
| 500 | 47 | 2592 | 61,0% | 81,8% |
| 1000 | 40 | 2262 | 51,9% | 71,4% |
| 2000 | 31 | 1854 | 40,3% | 58,5% |
| 4000 | 33 | 1455 | 43,0% | 46% |

On observe que les intensités lumineuses diminuent quand on ajoute des quantités croissantes de tartrazine. L'effet intéressant est que le niveau de luminosité du fond diminue plus rapidement que le niveau du signal du spot de référence. On considère la luminosité du fond du puits comme indésirable, générant un bruit dans la mesure du signal que l'on cherche à quantifier. On peut donc conclure que le rapport signal sur bruit est amélioré en ajoutant de la tartrazine.

**Tableau 2 : Intensité du signal et niveau de luminosité**

| **Concentration en tartrazine (µg/ml)** | **Intensité du fond du puits (en RLU)** | **Intensité du spot de référence (en RLU)** | **Rapport intensité du spot de référence sur intensité du fond du puits** | **Amélioration du rapport signal sur bruit (%)** |
|---|---|---|---|---|
| 0 | 77 | 3168 | 41,14 | 0,0% |
| 250 | 53 | 2784 | 52,53 | 27,7% |
| 500 | 47 | 2592 | 55,15 | 34,0% |
| 1000 | 40 | 2262 | 56,55 | 37,4% |
| 2000 | 31 | 1854 | 59,81 | 45,4% |
| 4000 | 33 | 1455 | 44,09 | 7,2% |

Le tableau 2 et la figure 10 montrent l'amélioration du rapport signal détecté sur bruit de fond quand on augmente la concentration de tartrazine. Un optimum semble exister à une concentration inférieure ou égale à 2000 µg/ml de tartrazine. Au-delà de cette concentration, le signal est très atténué et l'intérêt d'ajouter de la tartrazine pour améliorer la mesure du signal s'estompe.

### (ii) Détection de spots en fluorescence en présence de colorant

Il a également été vérifié que le signal émis par un fluorophore présent en tant que contrôle dans les spots d'une microplaque est bien détecté en présence colorant.

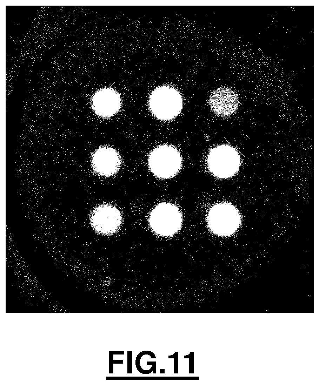

Comme on peut le voir sur la figure 11, en présence d'une solution absorbante comprenant de la tartrazine (utilisée à une concentration de 250µg/ml), le signal détecté en fluorescence permet de définir très nettement la position des spots par rapport au fond du puits. L'ajout de la solution absorbante n'empêche donc pas la détection du signal émis en fluorescence par un fluorophore présent dans les spots en tant que contrôle.

## Revendications

1. Procédé d'analyse multiplexe permettant d'améliorer la détection d'un signal correspondant à la présence d'un analyte comprenant les étapes suivantes :
a) fournir un support solide comprenant au moins deux compartiments, lesdits compartiments comprenant au moins deux spots destinés à la détection d'un analyte,
b) mettre un échantillon à analyser en présence des spots d'un compartiment,
c) mettre en présence des spots dudit compartiment au moins un ligand de détection d'un analyte, ledit ligand de détection d'un analyte étant couplé à un marqueur de détection direct ou indirect,
d) lorsque ledit marqueur de détection est un marqueur indirect, mettre en présence des spots dudit compartiment un rapporteur du marqueur de détection indirect couplé audit ligand de détection,
e) lorsque le rapporteur utilisé à l'étape d) est couplé à un marqueur indirect, mettre en présence des spots dudit compartiment un rapporteur du marqueur de détection indirect couplé audit rapporteur,
f) mettre au moins un colorant en présence des spots dudit compartiment, ledit ou lesdits colorants étant compris dans une phase liquide en contact des spots dudit compartiment et ledit au moins un colorant étant la tartrazine, et
g) détecter un signal correspondant à la présence d'un analyte au niveau des spots dudit compartiment, en présence de la phase liquide comprenant ledit ou lesdits colorants,
ledit procédé comprenant en outre une ou plusieurs étapes de lavage permettant d'éliminer les composés non liés aux spots ou aux différents composés liés directement ou indirectement aux spots,
aucune étape de lavage n'étant effectuée entre l'étape f) et l'étape g).

2. Procédé selon la revendication 1, **caractérisé en ce que** le signal est détecté à l'étape g) par le dessous du support solide.

3. Procédé selon la revendication 1 ou 2, **caractérisé en ce que** le signal détecté à l'étape g) est le signal émis par un composé luminescent et/ou le signal émis par un fluorophore.

4. Procédé selon l'une des revendications 1 à 3, **caractérisé en ce que** le colorant est apporté sous la forme d'une composition absorbante comprenant au moins un composé sélectionné dans le groupe consistant en du luminol, de l'isoluminol, un dérivé du luminol ou de l'isoluminol, un médiateur d'électrons, un oxydant et un solvant, par exemple de l'eau.

5. Utilisation d'au moins un colorant pour améliorer la détection d'un signal correspondant à la présence d'un analyte dans un procédé d'analyse multiplexe sur un support solide comprenant aux moins deux compartiments, lesdits compartiments comprenant au moins deux spots, ledit au moins un colorant étant la tartrazine et ledit procédé comprenant une ou plusieurs étapes de lavage permettant d'éliminer les composés non liés aux spots ou aux différents composés liés directement ou indirectement aux spots.

6. Utilisation selon la revendication 5, **caractérisée en ce que** la détection d'un signal correspondant à la présence d'un analyte est réalisée en présence d'une phase liquide.

7. Utilisation selon la revendication 5 ou 6, **caractérisée en ce que** le rapport signal détecté sur bruit de fond est augmenté.

8. Utilisation selon l'une des revendications 5 à 7, **caractérisée en ce que** le signal correspondant à la présence d'un analyte est émis par un composé chimiluminescent et/ou un fluorophore.

## Patentansprüche

1. Multiplex-Analyseverfahren zum Verbessern der Detektion eines Signals, das der Anwesenheit eines Analyten entspricht, das die folgenden Schritte aufweist:
a) Bereitstellen eines festen Trägers, der mindestens zwei Kammern aufweist, wobei die Kammern mindestens zwei Fleckstellen aufweisen, die zur Detektion eines Analyten bestimmt sind,
b) Platzieren einer zu analysierenden Probe in Gegenwart der Fleckstellen einer Kammer,
c) Platzieren mindestens eines Detektionsliganden eines Analyten in Gegenwart der Fleckstellen der Kammer, wobei der Detektionsligand eines Analyten an einen direkten oder indirekten Detektionsmarker gekoppelt ist,
d) wenn der Detektionsmarker ein indirekter Marker ist, Platzieren eines Reporters des an den Detektionsliganden gekoppelten indirekten Detektionsmarkers in Gegenwart der Fleckstellen der Kammer,
e) wenn der in Schritt d) verwendete Reporter an einen indirekten Marker gekoppelt ist, Platzieren eines Reporters des an den Reporter gekoppelten indirekten Detektionsmarkers in Gegenwart der Fleckstellen der Kammer,
f) Platzieren mindestens eines Farbstoffs in Gegenwart der Fleckstellen der Kammer, wobei der eine oder die mehreren Farbstoffe in einer flüssigen Phase enthalten sind, die in Kontakt mit den Fleckstellen der Kammer ist, und mindestens einer der Farbstoffe Tartrazin ist, und
g) Detektion eines Signals, das der Anwesenheit eines Analyten an den Fleckstellen der Kammer entspricht, in Gegenwart der flüssigen Phase, die den einen oder die mehreren Farbstoffe aufweist,
wobei das Verfahren ferner einen oder mehrere Schritte des Waschens aufweist, die das Entfernen der Verbindungen ermöglicht, die mit den Fleckstellen oder mit mit den Fleckstellen direkt oder indirekt verbundenen unterschiedlichen Verbindungen nicht verbunden sind,
wobei kein Schritt des Waschens zwischen dem Schritt f) und dem Schritt g) durchgeführt wird.

2. Verfahren nach Anspruch 1, **dadurch gekennzeichnet, dass** in Schritt g) das Signal von unterhalb des festen Trägers detektiert wird.

3. Verfahren nach Anspruch 1 oder 2, **dadurch gekennzeichnet, dass** das in Schritt g) detektierte Signal das von einer lumineszierenden Verbindung abgegebene Signal ist und / oder das von einem Fluorophor abgegebene Signal ist.

4. Verfahren nach einem der Ansprüche 1 bis 3, **dadurch gekennzeichnet, dass** der Farbstoff in Form einer absorbierenden Zusammensetzung bereitgestellt wird, der mindestens eine Verbindung ausgewählt aus der Gruppe bestehend aus Luminol, Isoluminol, einem Luminol- oder Isoluminolderivat, einem Elektronenmediator, einem Oxidationsmittel und einem Lösungsmittel, beispielsweise Wasser, aufweist.

5. Verwendung mindestens eines Farbstoffs zur Verbesserung der Detektion eines Signals, das der Anwesenheit eines Analyten entspricht, in einem Multiplex-Analyseverfahren auf einem festen Träger, der mindestens zwei Kammern aufweist, wobei die Kammern mindestens zwei Fleckstellen aufweisen, wobei der mindestens eine Farbstoff Tartrazin ist und das Verfahren einen oder mehrere Waschschritte aufweist, die das Entfernen der Verbindungen ermöglicht, die mit den Fleckenstellen oder mit mit den Fleckstellen direkt oder indirekt verbundenen unterschiedlichen Verbindungen nicht verbunden sind.

6. Verwendung nach Anspruch 5, **dadurch gekennzeichnet, dass** das Detektieren eines Signals, das der Anwesenheit eines Analyten entspricht, in Gegenwart einer flüssigen Phase erfolgt.

7. Verwendung nach Anspruch 5 oder 6, **dadurch gekennzeichnet, dass** das Verhältnis zwischen dem detektierten Signal und dem Hintergrundgeräusch erhöht ist.

8. Verwendung nach einem der Ansprüche 5 bis 7, **dadurch gekennzeichnet, dass** das Signal, das der Anwesenheit eines Analyten entspricht, von einer chemolumineszierenden Verbindung und / oder von einem Fluorophor abgegeben wird.

## Claims

1. Multiplex analysis method allowing the detection of a signal corresponding to the presence of an analyte to be improved, comprising the following steps:
a) providing a solid support comprising at least two compartments, said compartments comprising at least two spots for detection of an analyte,
b) bringing a sample to be analysed into contact with the spots of a compartment,
c) bringing at least one ligand for detection of an analyte into contact with the spots of said compartment, said ligand for detection of an analyte being coupled to a direct or indirect detection marker,
d) when said detection marker is an indirect marker, bringing a reporter of the indirect detection marker coupled to said detection ligand into contact with the spots of said compartment,
e) when the reporter used in step d) is coupled to an indirect marker, bringing a reporter of the indirect detection marker coupled to said reporter into contact with the spots of said compartment,
f) bringing at least one dye into contact with the spots of said compartment, said dye or dyes being contained in a liquid phase in contact with the spots of said compartment and said at least one dye being tartrazine, and
g) detecting a signal corresponding to the presence of an analyte in the region of the spots of said compartment, in the presence of the liquid phase comprising said dye or dyes,
said method further comprising one or more washing steps allowing the compounds that are not bound to the spots or to the various compounds bound directly or indirectly to the spots to be removed,
no washing step being carried out between step f) and step g).

2. Method according to claim 1, **characterised in that** the signal is detected in step g) through the bottom of the solid support.

3. Method according to claim 1 or 2, **characterised in that** the signal detected in step g) is the signal emitted by a luminescent compound and/or the signal emitted by a fluorophore.

4. Method according to any one of claims 1 to 3, **characterised in that** the dye is supplied in the form of an absorbent composition comprising at least one compound selected from the group consisting of luminol, isoluminol, a derivative of luminol or isoluminol, an electron mediator, an oxidant and a solvent, for example water.

5. Use of at least one dye for improving the detection of a signal corresponding to the presence of an analyte in a multiplex analysis method on a solid support comprising at least two compartments, said compartments comprising at least two spots, said at least one dye being tartrazine and said method comprising one or more washing steps allowing the compounds that are not bound to the spots or to the various compounds bound directly or indirectly to the spots to be removed.

6. Use according to claim 5, **characterised in that** the detection of a signal corresponding to the presence of an analyte is carried out in the presence of a liquid phase.

7. Use according to claim 5 or 6, **characterised in that** the detected signal to background noise ratio is increased.

8. Use according to any one of claims 5 to 7, **characterised in that** the signal corresponding to the presence of an analyte is emitted by a chemiluminescent compound and/or a fluorophore.
